# EUROPEAN PATENT APPLICATION

(11) **EP 3 505 529 A1**
(43) Date of publication of application: **03.07.2019**
(21) Application number: 17844002.0
(22) Date of filing: 28.08.2017
(51) Int. Cl.: C07K 7/06, C07K 7/08, A61K 38/08, A61K 38/10

(54) **PEPTIDE FOR PROMOTING OSTEOANAGENESIS OR OSTEOGENESIS AND USE OF SAME**

(30) Priority: 26.08.2016 KR 20160109232
(71) Applicant: Sewon Biotechnology Inc., Seoul 03080 (KR)
(72) Inventor: YOON, Won-Joon, Seoul 01063 (KR)
(74) Representative: Ter Meer Steinmeister & Partner
(86) International application number: PCT/KR2017/009373
(87) International publication number: WO 2018/038582

(57) **Abstract**

The present application discloses a peptide for promoting osteoanagenesis or osteogenesis, and a composition including same. The peptide or the composition including the same according to the present application has osteoanagenesis and osteogenesis functions in heterogenous bones and allogenic bones and promotes osteogenesis in a generation process too. In addition, the peptide or the composition does not exhibit heterotropic ossification. Accordingly, the composition including the peptide according to the present application can be applied to treatments of various diseases requiring osteoanagenesis and osteogenesis functions. Moreover, the peptide according to the present application can ensure safety by replacing BMP-2 or by being used with small quantities of BMP-2.

<110>     YOON, WON JOON

<120>     Peptide stimulating bone regeneration or bone formation and its
          use

<130>     PCT201708004

<150>     KR 2016-0109232
<151>     2016-08-26

<160>     79

<170>     KopatentIn 2.0

<210>     1
<211>     5
<212>     PRT
<213>     Artificial Sequence

<220>
<223>     artificial


<400>     1
Gln Leu Val Val Lys
  1               5


<210>     2
<211>     5
<212>     PRT
<213>     Artificial Sequence

<220>
<223>     artificial


<400>     2
Gln Glu Glu Glu Lys
  1               5


<210>     3
<211>     5
<212>     PRT
<213>     Artificial Sequence

<220>
<223>     artificial


<400>     3
Gln Ala Ala Ala Lys
  1               5


<210>     4
<211>     5
<212>     PRT
<213>     Artificial Sequence

<220>
<223>     artificial

<400>    4
Asn Leu Val Val Lys
1               5

<210>    5
<211>    5
<212>    PRT
<213>    Artificial Sequence

<220>
<223>    artificial

<400>    5
Ser Leu Val Val Lys
1               5

<210>    6
<211>    6
<212>    PRT
<213>    Artificial Sequence

<220>
<223>    artificial

<400>    6
Phe Arg Ala Leu Pro Cys
1               5

<210>    7
<211>    6
<212>    PRT
<213>    Artificial Sequence

<220>
<223>    artificial

<400>    7
Phe Arg Glu Glu Pro Cys
1               5

<210>    8
<211>    6
<212>    PRT
<213>    Artificial Sequence

<220>
<223>    artificial

<400>    8
Phe Arg Val Val Pro Cys
1               5

<210>    9
<211>    6
<212>    PRT
<213>    Artificial Sequence

<220>
<223>    artificial


<400>    9
Phe Glu Ala Leu Pro Cys
1               5


<210>    10
<211>    6
<212>    PRT
<213>    Artificial Sequence

<220>
<223>    artificial


<400>    10
Tyr Arg Ala Leu Pro Cys
1               5


<210>    11
<211>    6
<212>    PRT
<213>    Artificial Sequence

<220>
<223>    artificial


<400>    11
Trp Arg Ala Leu Pro Cys
1               5


<210>    12
<211>    5
<212>    PRT
<213>    Artificial Sequence

<220>
<223>    artificial


<400>    12
Phe Arg Ala Leu Pro
1               5


<210>    13
<211>    4
<212>    PRT
<213>    Artificial Sequence

<220>
<223>    artificial

<400>    13
Phe Arg Ala Leu
  1

<210>    14
<211>    4
<212>    PRT
<213>    Artificial Sequence

<220>
<223>    artificial

<400>    14
Phe Arg Pro Cys
  1

<210>    15
<211>    6
<212>    PRT
<213>    Artificial Sequence

<220>
<223>    artificial

<400>    15
Arg Gln Leu Val Val Lys
  1               5

<210>    16
<211>    12
<212>    PRT
<213>    Artificial Sequence

<220>
<223>    artificial

<400>    16
Phe Arg Ala Leu Pro Cys Arg Gln Leu Val Val Lys
  1               5                   10

<210>    17
<211>    12
<212>    PRT
<213>    Artificial Sequence

<220>
<223>    artificial

<400>    17
Arg Gln Leu Val Val Lys Phe Arg Ala Leu Pro Cys
  1               5                   10

<210>    18
<211>    24
<212>    PRT
<213>    Artificial Sequence

<220>
<223>    artificial


<400>    18
Arg Gln Leu Val Val Lys Phe Arg Ala Leu Pro Cys Arg Gln Leu Val
1               5                   10                  15

Val Lys Phe Arg Ala Leu Pro Cys
            20


<210>    19
<211>    11
<212>    PRT
<213>    Artificial Sequence

<220>
<223>    artificial


<400>    19
Arg Gln Leu Val Val Lys Phe Arg Ala Leu Pro
1               5                   10


<210>    20
<211>    10
<212>    PRT
<213>    Artificial Sequence

<220>
<223>    artificial


<400>    20
Arg Gln Leu Val Val Lys Phe Arg Ala Leu
1               5                   10


<210>    21
<211>    12
<212>    PRT
<213>    Artificial Sequence

<220>
<223>    artificial


<400>    21
Lys Gln Leu Val Val Lys Phe Arg Ala Leu Pro Cys
1               5                   10


<210>    22
<211>    9
<212>    PRT
<213>    Artificial Sequence

<220>
<223>    artificial

<400>    22
Arg Gln Lys Phe Arg Ala Leu Pro Cys
1               5

<210>    23
<211>    12
<212>    PRT
<213>    Artificial Sequence

<220>
<223>    artificial

<400>    23
Arg Gln Glu Glu Glu Lys Phe Arg Ala Leu Pro Cys
1               5                   10

<210>    24
<211>    12
<212>    PRT
<213>    Artificial Sequence

<220>
<223>    artificial

<400>    24
Arg Gln Ala Ala Ala Lys Phe Arg Ala Leu Pro Cys
1               5                   10

<210>    25
<211>    10
<212>    PRT
<213>    Artificial Sequence

<220>
<223>    artificial

<400>    25
Arg Gln Leu Val Val Lys Phe Arg Pro Cys
1               5                   10

<210>    26
<211>    12
<212>    PRT
<213>    Artificial Sequence

<220>
<223>    artificial

<400>    26
Arg Gln Leu Val Val Lys Phe Arg Glu Glu Pro Cys

1                   5                   10

<210>   27
<211>   12
<212>   PRT
<213>   Artificial Sequence

<220>
<223>   artificial

<400>   27
Arg Gln Leu Val Val Lys Phe Arg Val Val Pro Cys
1               5                   10

<210>   28
<211>   12
<212>   PRT
<213>   Artificial Sequence

<220>
<223>   artificial

<400>   28
Arg Gln Glu Glu Glu Lys Phe Arg Glu Glu Pro Cys
1               5                   10

<210>   29
<211>   12
<212>   PRT
<213>   Artificial Sequence

<220>
<223>   artificial

<400>   29
Glu Gln Leu Val Val Glu Phe Glu Ala Leu Pro Cys
1               5                   10

<210>   30
<211>   12
<212>   PRT
<213>   Artificial Sequence

<220>
<223>   artificial

<400>   30
Arg Gln Leu Val Val Lys Tyr Arg Ala Leu Pro Cys
1               5                   10

<210>   31
<211>   12
<212>   PRT
<213>   Artificial Sequence

<220>
<223>    artificial


<400>    31
Arg Gln Leu Val Val Lys Trp Arg Ala Leu Pro Cys
1               5                   10


<210>    32
<211>    12
<212>    PRT
<213>    Artificial Sequence

<220>
<223>    artificial


<400>    32
Arg Asn Leu Val Val Lys Phe Arg Ala Leu Pro Cys
1               5                   10


<210>    33
<211>    12
<212>    PRT
<213>    Artificial Sequence

<220>
<223>    artificial


<400>    33
Arg Ser Leu Val Val Lys Phe Arg Ala Leu Pro Cys
1               5                   10


<210>    34
<211>    16
<212>    PRT
<213>    Artificial Sequence

<220>
<223>    artificial


<400>    34
Arg Gln Leu Val Val Gln Leu Val Val Lys Phe Arg Ala Leu Pro Cys
1               5                   10                  15


<210>    35
<211>    20
<212>    PRT
<213>    Artificial Sequence

<220>
<223>    artificial


<400>    35

Arg Gln Leu Val Val Gln Leu Val Val Gln Leu Val Val Lys Phe Arg
1               5                   10                  15

Ala Leu Pro Cys
            20

<210>   36
<211>   24
<212>   PRT
<213>   Artificial Sequence

<220>
<223>   artificial

<400>   36
Arg Gln Leu Val Val Gln Leu Val Val Gln Leu Val Val Gln Leu Val
1               5                   10                  15

Val Lys Phe Arg Ala Leu Pro Cys
            20

<210>   37
<211>   18
<212>   PRT
<213>   Artificial Sequence

<220>
<223>   artificial

<400>   37
Arg Gln Leu Val Val Lys Phe Arg Ala Leu Pro Cys Phe Arg Ala Leu
1               5                   10                  15

Pro Cys

<210>   38
<211>   13
<212>   PRT
<213>   Artificial Sequence

<220>
<223>   artificial

<400>   38
Arg Arg Gln Leu Val Val Lys Phe Arg Ala Leu Pro Cys
1               5                   10

<210>   39
<211>   16
<212>   PRT
<213>   Artificial Sequence

<220>
<223>   artificial

<400>    39
Arg Arg Arg Arg Arg Gln Leu Val Val Lys Phe Arg Ala Leu Pro Cys
1               5                   10                  15

<210>    40
<211>    21
<212>    PRT
<213>    Artificial Sequence

<220>
<223>    artificial

<400>    40
Arg Arg Arg Arg Arg Arg Arg Arg Arg Arg Gln Leu Val Val Lys Phe
1               5                   10                  15

Arg Ala Leu Pro Cys
            20

<210>    41
<211>    26
<212>    PRT
<213>    Artificial Sequence

<220>
<223>    artificial

<400>    41
Arg Arg Arg Arg Arg Arg Arg Arg Arg Arg Arg Arg Arg Arg Arg Gln
1               5                   10                  15

Leu Val Val Lys Phe Arg Ala Leu Pro Cys
            20                  25

<210>    42
<211>    12
<212>    PRT
<213>    Artificial Sequence

<220>
<223>    artificial

<400>    42
Arg Gln Val Val Val Lys Phe Arg Ala Leu Pro Cys
1               5                   10

<210>    43
<211>    12
<212>    PRT
<213>    Artificial Sequence

<220>
<223>    artificial

<400>    43
Arg Gln Ile Val Val Lys Phe Arg Ala Leu Pro Cys
1               5                   10

<210>    44
<211>    12
<212>    PRT
<213>    Artificial Sequence

<220>
<223>    artificial

<400>    44
Arg Gln Ala Val Val Lys Phe Arg Ala Leu Pro Cys
1               5                   10

<210>    45
<211>    12
<212>    PRT
<213>    Artificial Sequence

<220>
<223>    artificial

<400>    45
Arg Gln Glu Val Val Lys Phe Arg Ala Leu Pro Cys
1               5                   10

<210>    46
<211>    12
<212>    PRT
<213>    Artificial Sequence

<220>
<223>    artificial

<400>    46
Arg Gln Leu Leu Val Lys Phe Arg Ala Leu Pro Cys
1               5                   10

<210>    47
<211>    12
<212>    PRT
<213>    Artificial Sequence

<220>
<223>    artificial

<400>    47
Arg Gln Leu Ile Val Lys Phe Arg Ala Leu Pro Cys
1               5                   10

<210>    48

<211>    12
<212>    PRT
<213>    Artificial Sequence

<220>
<223>    artificial


<400>    48
Arg Gln Leu Ala Val Lys Phe Arg Ala Leu Pro Cys
1               5                   10


<210>    49
<211>    12
<212>    PRT
<213>    Artificial Sequence

<220>
<223>    artificial


<400>    49
Arg Gln Leu Glu Val Lys Phe Arg Ala Leu Pro Cys
1               5                   10


<210>    50
<211>    12
<212>    PRT
<213>    Artificial Sequence

<220>
<223>    artificial


<400>    50
Arg Gln Leu Val Leu Lys Phe Arg Ala Leu Pro Cys
1               5                   10


<210>    51
<211>    12
<212>    PRT
<213>    Artificial Sequence

<220>
<223>    artificial


<400>    51
Arg Gln Leu Val Ile Lys Phe Arg Ala Leu Pro Cys
1               5                   10


<210>    52
<211>    12
<212>    PRT
<213>    Artificial Sequence

<220>
<223>    artificial

<400>    52
Arg Gln Leu Val Ala Lys Phe Arg Ala Leu Pro Cys
1               5                   10

<210>    53
<211>    12
<212>    PRT
<213>    Artificial Sequence

<220>
<223>    artificial

<400>    53
Arg Gln Leu Val Glu Lys Phe Arg Ala Leu Pro Cys
1               5                   10

<210>    54
<211>    12
<212>    PRT
<213>    Artificial Sequence

<220>
<223>    artificial

<400>    54
Arg Gln Leu Val Val Arg Phe Arg Ala Leu Pro Cys
1               5                   10

<210>    55
<211>    12
<212>    PRT
<213>    Artificial Sequence

<220>
<223>    artificial

<400>    55
Arg Gln Leu Val Val Lys Phe Lys Ala Leu Pro Cys
1               5                   10

<210>    56
<211>    12
<212>    PRT
<213>    Artificial Sequence

<220>
<223>    artificial

<400>    56
Arg Gln Leu Val Val Glu Phe Glu Ala Leu Pro Cys
1               5                   10

<210>    57

<211>    12
<212>    PRT
<213>    Artificial Sequence

<220>
<223>    artificial


<400>    57
Arg Gln Leu Val Val Lys Phe Arg Leu Leu Pro Cys
1               5                   10


<210>    58
<211>    12
<212>    PRT
<213>    Artificial Sequence

<220>
<223>    artificial


<400>    58
Arg Gln Leu Val Val Lys Phe Arg Ile Leu Pro Cys
1               5                   10


<210>    59
<211>    12
<212>    PRT
<213>    Artificial Sequence

<220>
<223>    artificial


<400>    59
Arg Gln Leu Val Val Lys Phe Arg Val Leu Pro Cys
1               5                   10


<210>    60
<211>    12
<212>    PRT
<213>    Artificial Sequence

<220>
<223>    artificial


<400>    60
Arg Gln Leu Val Val Lys Phe Arg Glu Leu Pro Cys
1               5                   10


<210>    61
<211>    12
<212>    PRT
<213>    Artificial Sequence

<220>
<223>    artificial

<400>    61
Arg Gln Leu Val Val Lys Phe Arg Ala Ala Pro Cys
1               5                   10

<210>    62
<211>    12
<212>    PRT
<213>    Artificial Sequence

<220>
<223>    artificial

<400>    62
Arg Gln Leu Val Val Lys Phe Arg Ala Ile Pro Cys
1               5                   10

<210>    63
<211>    12
<212>    PRT
<213>    Artificial Sequence

<220>
<223>    artificial

<400>    63
Arg Gln Leu Val Val Lys Phe Arg Ala Val Pro Cys
1               5                   10

<210>    64
<211>    12
<212>    PRT
<213>    Artificial Sequence

<220>
<223>    artificial

<400>    64
Arg Gln Leu Val Val Lys Phe Arg Ala Glu Pro Cys
1               5                   10

<210>    65
<211>    12
<212>    PRT
<213>    Artificial Sequence

<220>
<223>    artificial

<400>    65
Arg Gln Glu Glu Glu Glu Phe Glu Glu Glu Pro Cys
1               5                   10

<210>    66

<211>    12
<212>    PRT
<213>    Artificial Sequence

<220>
<223>    artificial


<220>
<221>    VARIANT
<222>    (11)
<223>    any amino acids


<400>    66
Arg Gln Leu Val Val Lys Phe Arg Ala Leu Xaa Cys
1               5                   10


<210>    67
<211>    12
<212>    PRT
<213>    Artificial Sequence

<220>
<223>    artificial


<400>    67
Arg Gln Leu Val Val Lys Phe Arg Ala Leu Pro Ser
1               5                   10


<210>    68
<211>    12
<212>    PRT
<213>    Artificial Sequence

<220>
<223>    artificial


<400>    68
Arg Gln Leu Val Val Lys Phe Arg Ala Leu Pro Thr
1               5                   10


<210>    69
<211>    12
<212>    PRT
<213>    Artificial Sequence

<220>
<223>    artificial


<220>
<221>    VARIANT
<222>    (12)
<223>    any amino acids


<400>    69

Arg Gln Leu Val Val Lys Phe Arg Ala Leu Pro Xaa
1               5                   10

<210>   70
<211>   5
<212>   PRT
<213>   Artificial Sequence

<220>
<223>   artificial

<400>   70
Gln Val Val Val Lys
1               5

<210>   71
<211>   5
<212>   PRT
<213>   Artificial Sequence

<220>
<223>   artificial

<400>   71
Gln Ile Val Val Lys
1               5

<210>   72
<211>   5
<212>   PRT
<213>   Artificial Sequence

<220>
<223>   artificial

<400>   72
Gln Ala Val Val Lys
1               5

<210>   73
<211>   5
<212>   PRT
<213>   Artificial Sequence

<220>
<223>   artificial

<400>   73
Gln Leu Leu Val Lys
1               5

<210>   74
<211>   5
<212>   PRT

<213>    Artificial Sequence

<220>
<223>    artificial

<400>    74
Gln Leu Ile Val Lys
1               5

<210>    75
<211>    5
<212>    PRT
<213>    Artificial Sequence

<220>
<223>    artificial

<400>    75
Gln Leu Ala Val Lys
1               5

<210>    76
<211>    5
<212>    PRT
<213>    Artificial Sequence

<220>
<223>    artificial

<400>    76
Gln Leu Val Leu Lys
1               5

<210>    77
<211>    5
<212>    PRT
<213>    Artificial Sequence

<220>
<223>    artificial

<400>    77
Gln Leu Val Ile Lys
1               5

<210>    78
<211>    5
<212>    PRT
<213>    Artificial Sequence

<220>
<223>    artificial

<400>    78

Gln Leu Val Ala Lys
1               5

<210>    79
<211>    5
<212>    PRT
<213>    Artificial Sequence

<220>
<223>    artificial

<400>    79
Gln Leu Val Val Arg
1               5

## Description

### BACKGROUND OF THE INVENTION

### Field of the Invention

The present disclosure relates to a technology that can promote the bone regeneration and bone formation.

### Description of the Related Art

By 2050, it is expected that the population aged 60 or older account for 21.8% of the population, and thus, aging, metabolic diseases, and other skeletal diseases will continue to increase.

It is known that the fusion rate of the injured area can be very low when the fracture occurs in the elderly and osteoporosis patients, and in the case of osteoporosis patients older than 70 years, it is known that the fracture causes a very high mortality due to the deterioration of the quality of life

Thus, medical technology that can cure fractures effectively and quickly has become a major social issue. When bone tissue is affected by infections, trauma, and diseases, and the rate of natural healing become very slow. As the aging progresses, the rate decreases further. Thus rapid and accurate recovery and healing based on bone regeneration are more important than any disease considering the pain the patient suffers.

In Korea alone, the annual number of patients requiring bone regeneration therapy is estimated to reach 2 million, and the related health insurance and medical expenses are estimated to exceed 1 trillion won, and the socioeconomic loss due to bone loss is expected to be much larger.

Therefore, the development of bone graft materials capable of effectively inducing regeneration of bone tissue is of great value in terms of health and socioeconomic aspects (Dimitriou et al., BMC Medicine 2011, 9:66).

For effective bone regeneration, osteoconductive materials, osteogenic materials, and osteoinductive materials are required.

As the bone conduction material, a substance capable of providing an environment similar to bone such as calcium hydroxyphosphate having a particular mechanical strength is used, and a bone-derived osteogenic cell population and an osteogenic bioactive factor are used together.

WO 2009-093240 discloses erythropoietin and fibronectin compositions for bone regeneration. BMP-2, 4, and 7 proteins are currently the most widely used osteogenic bioactive factors. In particular, BMP-2 has been approved by the FDA in 2002 for the purpose of cervical fusion, lumbar fusion, and alveolar bone regeneration. However, many side effects such as the following are pointed out as problems. For example, inflammatory side effects lead to pseudarhrosis, repressed bone formation due to bone resorption (rather aggravates bone mineral density), delayed wound healing, and pain, which are typical side effects found in 85% of BMP-2 treated patients. Ectopic ossification is another irreversible fatal side effect causing muscle calcification, injection side effects, or airway obstruction (cervical surgery patients, death). In addition, injection side effects occur at 1.5% of all patients. It is known that more than about 20 kinds of cancers including rare cancers occur within 24 months when using 5mg or more, and it occurs in 5% of the group. In the dental field, it is known that side effects on pain are severe.

Therefore, BMP-2 is considered to have a limitation in further application due to the seriousness of its side effects. As a result, it is expected that the social and economic effect of the technology that can overcome or replace the safety and efficacy of BMP-2 side effects is very large. However, effective and potent drug candidates have not been developed at present.

### SUMMARY OF THE INVENTION

The present disclosure is to provide a composition or a method that can effectively promote bone formation or bone regeneration without side effects.

In one aspect, the present disclosure provides a use of a polypeptide having the formula IV below for bone regeneration or bone formation:

[X¹²]₁₋₁₅-[X¹-X²-X³-X⁴-X⁵]ₘ-[X⁶-X⁷-X⁸-X⁹-X¹⁰-X¹¹]ₙ,

wherein
in the -[X¹-X²-X³-X⁴-X⁵]ₘ,
   X¹ is any amino acid,
   X², X³ and X⁴, which may be identical or different, are each L, V, I, E, G or A, at least one of X², X³ and X⁴ may be absent, and
   X⁵ is K or R,
   m is an integer from 1 to 5, if m is 2 or more, each polypeptide may be identical or different;
in the [X⁶-X⁷-X⁸-X⁹-X¹⁰-X¹¹]ₙ,
   the [X⁶-X⁷-X⁸-X⁹-X¹⁰-X¹¹]ₙ may be absent, or
   X⁶ is any one of F, Y and W,
   X⁷ is K or R,
   X⁸ is any one of A, M and I
   X⁹ is any one of L, M and G,
   X¹⁰ is any amino acid, and
   X¹¹ is any one of C, S and T, wherein at least one group of (X⁸ and X⁹) and (X¹⁰ and X¹¹) may be absent, wherein n is 1 or 2, and
   X¹² is any one of F, K and R,
the amino acid residue is a synthetic or naturally occurring, D- or L-form, or derivatives thereof.

In one embodiment, X¹ is any one of S, T, C, P, N and Q.

In other embodiment, X²-X³-X⁴ is AAA, EEE, LVG, LVA, LVL, LVV, LLA, LLL, or LLV.

In still other embodiment, the [X¹-X²-X³-X⁴-X⁵] has the amino acid sequence set forth in QLVVK (SEQ ID NO: 1), QEEEK (SEQ ID NO: 2), QAAAK (SEQ ID NO: 3), NLVVK (SEQ ID NO: 4)), or SLVVK (SEQ ID NO: 5), QVVVK (SEQ ID NO: 70), QIVVK (SEQ ID NO: 71), QAVVK (SEQ ID NO: 72), QLLVK (SEQ ID NO: 73), QLIVK (SEQ ID NO: 74), QLAVK (SEQ ID NO: 75), QLVLK (SEQ ID NO: 76), QLVIK (SEQ ID NO: 77), QLVAK (SEQ ID NO: 78), or QLVVR (SEQ ID NO: 79).

In still other embodiment, the [X⁶-X⁷-X⁸-X⁹-X¹⁰-X¹¹]ₙ has the amino acid sequence set forth in FRALPC (SEQ ID NO: 6), FREEPC (SEQ ID NO: 7), FRVVPC (SEQ ID NO: 8), FEALPC (SEQ ID NO: 9), YRALPC (SEQ ID NO: 10), WRALPC (SEQ ID NO: 11), FRALP (SEQ ID NO: 12), FRAL(SEQ ID NO: 13), FRPC (SEQ ID NO: 14) or FR.

In still other embodiment, at least two molecules of the polypeptide of the present disclosure may be linked to together.

In still other embodiment, the present peptide has the amino acid sequence set forth in SEQ ID NO: 15 to 33 or 37 to 69.

In still other embodiment, the peptide may further comprise 5 to 20 K or R residues at its N-terminus.

In other aspect, the present peptide of formula IV for bone regeneration or bone formation in which X¹ is polar Q, N, S, T, P or C; X², X³ and X⁴, which may be identical or different, are each hydrophobic L, V, I, or A; X⁵ is positively charged K or R, and in the [X⁶-X⁷-X⁸-X⁹-X¹⁰-X¹¹]ₙ, at least one group of (X⁸ and X⁹) and (X¹⁰ and X¹¹) may be absent, and has the amino acid sequence set forth by FRALPC (SEQ ID NO: 6), FREEPC (SEQ ID NO: 7), FRVVPC (SEQ ID NO: 8), FEALPC (SEQ ID NO: 9), YRALPC (SEQ ID NO: 10), WRALPC (SEQ ID NO: 11), FRALP (SEQ ID NO: 12), FRAL(SEQ ID NO: 13), FRPC (SEQ ID NO: 14) or FR; X¹² is positively charged K or R,; m and n are each 1 or 2, when m or n is 2, each amino acid sequence may be same or different, the amino acid is a synthetic or naturally occurring, D- or L-form.

In one embodiment, X²-X³-X⁴ is LVV, AAA, LVA, LVL, LLA, LLL, or LLV.

In still other embodiment, the [X¹-X²-X³-X⁴-X⁵] has the amino acid sequence represented by any one of the following: QLVVK (SEQ ID NO: 1), QAAAK (SEQ ID NO: 3), NLVVK (SEQ ID NO: 4), SLVVK (SEQ ID NO: 5), QVVVK (SEQ ID NO: 70), QIVVK (SEQ ID NO: 71), QAVVK (SEQ ID NO: 72), QLLVK (SEQ ID NO: 73), QLIVK (SEQ ID NO: 74), QLAVK (SEQ ID NO: 75), QLVLK (SEQ ID NO: 76), QLVIK (SEQ ID NO: 77), QLVAK (SEQ ID NO: 78), and QLVVR (SEQ ID NO: 79).

In still other embodiment, the present peptide is represented by SEQ ID NO: 17, 19, 20, 21, 24, 25, 26, 27, 30, 31, 32, 33, 37, 38, 39, 40, 41, 42, 43, 44, 46, 47, 48, 50, 51, 52, or 54..

In one embodiment, the present peptide may further comprise 5 to 20 K or R residues at its N-terminus.

In other aspect, there is provided the peptides having the amino acid sequence represented by any one of SEQ ID NO: 15 to 69 or composition or pharmaceutical composition for promoting bone formation or bone regeneration.

In one embodiment, the N- or C-terminus of the peptides are substituted with non-reactive group.

The present peptides or the composition comprising the same are used for treating or preventing a disease that requires an increase in the bone density, the diseases comprise a fracture, alveolar bone regeneration, arthroplasty, spine fusion or bone cyst, without being limited thereto.

In other aspect, there is provided a method of promoting bone formation or bone regeneration by administering the present polypeptide or a composition comprising the same to a subject in need thereof to treat a disease that requires an increase in the bone density due to bone loss.

In other aspect, there is provided a use of the present peptide as described herein to treat or prevent a disease that requires an increase in the bone density particularly due to bone loss, and the disease includes a fracture, alveolar bone regeneration, arthroplasty, spine fusion or bone cyst, but is not limited thereto.

### ADVANTAGEOUS EFFECTS

The present peptides and the composition comprising the same can promote the bone formation and bone regeneration as well as promote the bone formation during the development. Also The present peptides and the composition comprising the same do not have the side effect to ectopic ossification. Thus the composition comprising the present peptides can be advantageously used to treat various diseases that requires the bone regeneration and/or bone formation. Also the present peptides may replace the use of BMP-2 or may be used in combination with a less concentration of BMP-2 than regularly used.

The present peptides show a comparable to or better than BMP-2 in terms of the rate of recovery. Thus the present peptides or the composition comprising the same can replace the BMP-2, are less expensive than BMP-2 and can be used with existing bone substitutes or bone grafts to effect the recovery of the bone tissue at a higher level than the conventional ones. In addition, it has also advantages in synthesis, storage and stability due to its simple chemical structures compared to other osteoinductive or osteoconductive bone substitute materials, etc. Further the present peptides with adhesion property to bone tissue can be advantageously used not only for surgical operation but also for administration by various routes such as local administration.

### BRIEF DESCRIPTION OF THE DRAWINGS

FIG. 1 is a quantitative analysis result of hard tissue formation formed by the peptide treatment according to the present invention in a mouse xenograft bone regeneration experiment. H&E staining was performed to determine the area of the hard tissue-specific high-density stained area. Control groups were treated with saline, and as comparative groups, BMP-2 alone and rhBMP-2 mixed with the present peptide were used.
FIG. 2 shows immunohistochemical staining results of the ossified tissues of the tissues regenerated in xenograft bone regeneration experiment using mouse. Immunostaining was performed using an antibody specific for Osteocalcin, a marker that is specifically expressed during the biological calcification of the hard tissue.
FIG. 3 shows the results of immunohistochemical staining using antibodies against CD31, which is a marker of blood cells and blood vessels, in the hard tissue regenerated in xenograft bone regeneration experiment using mouse. This result was performed to evaluate whether the tissue formed through the formation of blood vessels maintains its biological activity.
FIG. 4 shows the results of three-dimensional rendering images taken at 3 weeks and 5 weeks after treatment of the injured calvarial in the bone regeneration experiment using rat calvarial defect models. Control groups were treated with saline, as comparative groups, BMP-2 alone and rhBMP-2 mixed with the present peptide were used.
FIG. 5 is a result of quantitative measurement of the bone volume of the new bone tissue in the bone regeneration assay using a rat calvarial defect model.
FIG. 6 is a graph showing the results of quantitative analysis of the expression of various osteogenic factors and markers in the new bone tissue formed in the bone regeneration experiment using rat calvarial defect models.
FIG. 7 shows the results of analysis for investigating side effects related to ectopic ossification. BMP-2 was used at the left femoral muscle of the mouse, and the peptide of the present invention was used at the right femoral muscle. After 4 weeks, uCT analysis was performed to analyze the calcification of the muscle tissue using a three-dimensional rendering image.
FIG. 8 shows the results of micro-CT analysis of the bone formation promotion assay during the development.
FIG. 9 shows the result of whole skeletal staining (WSS) analysis of the bone formation promotion assay during the development.
FIG. 10 shows the results of histological analysis using H & E staining of the hindlimb of the embryos from each experimental group in the experiment to test the bone formation promotion during the development.
FIG. 11 shows the results of Alcian blue staining of the hindlimb of the embryos from each experimental group in the experiment to test the bone formation promotion during the development.
FIG. 12 is the result of analysis of surface adhesion to a hydrophobic surface of a cell, which is a significant biological mechanism for promoting bone formation, showing the changes in the adhesion according to the peptide concentration used. Quantitative analysis of the attached cells was performed by quantifying the DNA of cells attached to the surface.
FIG. 13 is the result of analysis of surface adhesion to a hydrophobic surface of a cell, which is a significant biological mechanism for promoting bone formation, showing the changes in the adhesion according to the time after the treatment of the present peptide.
FIG. 14 shows the results of a series of changes in the cell attachment form or shape on a rigid structural support having a high hardness such as bone by the treatment with the present peptide. The support used was titanium, which is used as a dental implant material.
FIG. 15 shows the effect of the present peptide on the area where the cells are attached on the rigid structure support having high hardness such as bone, analyzed with the confocal microscopic method, showing the promotion of the cell attachment by the present peptides. The supports used was orthopedic titanium with various strength and surface properties.
FIG. 16 is a result of the quantitative analysis of the adhesion promotion property of the presents by measuring the adhesion area occupied by the cells on a rigid structure support having high hardness such as bone.
FIG. 17 is a result of examining the distribution of surface distribution of the cells on a rigid structure support having high hardness such as bone. This is the result of using zirconia as a high hardness support.
FIG. 18 is a diagram illustrating the mechanism of action of the peptides of the present invention to induce bone regeneration, based on various analysis results shown in the present figures.
FIG. 19 is a result showing the effect of the present various peptides (horizontal axis) having the property of promoting cell attachment on promoting bone formation. It shows that osteogenesis of the fetus is promoted when the peptides was injected into a pregnant mouse. The horizontal axis represents SEQ ID NOs of the peptide used.

### DETAILED DESCRIPTION OF THE EMBODIMENTS

In one aspect, the present disclosure relates to a peptide or polypeptides or derivatives thereof having promoting bone regeneration and/or bone formation activity of Formula I: [X¹-X²-X³-X⁴-X⁵]ₘ :
in which
X¹ is any amino acid, or particularly with a polar non charged side chain,
X², X³ and X⁴, which may be identical or different, are each L, V, I, E G or A, and at least one of X², X³ and X⁴ may be absent,
X⁵ is a positively charged amino acid, and
wherein M is an integer from 1 to 5, if m is 2 or more, each polypeptide may be identical or different, wherein the amino acid is a natural or non-natural D- or L-form residue.

As used herein the term "amino acid" refers to naturally occurring 20 amino acids or non-natural amino acids, as well as post-translationally modified amino acids, amino acid analogs and amino acid mimetics that function in a manner similar to the naturally occurring amino acids, including for example, phosphoserine and phosphothreonine; rare amino acids such as 2-aminoadipic acid, hydroxylysine, norvaline and norleucine; amino acids modified to improve their stability in cell and cell penetration; and optical isomers of D- and L-form.

Positively charged amino acids, negatively charged amino acids, polar non-charged amino acids, and non-polar aliphatic amino acids, which may be used in the present disclosure, are well known in the art and may be selected by one of ordinary person in the art without difficulty.

In one embodiment of the present disclosure, positively charged amino acids are K or R.

In other embodiment of the present disclosure, negatively charged amino acids are D or E.

In still other embodiment of the present disclosure, polar non-charged amino acids are S, T, C, P, N or Q.

In still other embodiment of the present disclosure non-polar aliphatic amino acids are G, A, L, V, M or I.

As used herein, the terms natural and non-natural each refers to the compounds found in cells, tissues or bodies; and the compounds artificially modified thereto for a particular purpose, respectively.

As used herein, the term peptides and polypetpdies are interchangeably used, and are read from N- to C- term unless defined otherwise and refer to a molecule in which amino acid monomers are covalently linked to each other, and are interpreted to include peptides comprised of native amino acids or their lysed products, synthetic peptides, peptides prepared in a recombinant manner, peptide mimetics (typically synthetic peptide), analogous such as peptoide and sempeptoids, and peptides modified to improve/change their function such as stability in cells. Examples of the modification include a N-term modification, a C-term modification, a peptide bond modification such as CH₂-NH, CH₂-S, CH₂-S=O and CH₂-CH₂, a back-bone modification, and side-chain modification. Peptide mimetics are prepared by methods known in the art, which may be referred to, for example, Quantitative Drug Design, C.A. Ramsden Gd., Choplin Pergamon Press (1992).

In the present disclosure, the peptide of formula I, also referred as a first domain/region, is found to be involved in the adhesion to cell surfaces or cell membranes and also can be present in multiple numbers depending on the particular applications of interest as described herein. Particularly the first region is hydrophobic in nature enabling hydrophobic interactions between molecules of the surfaces, or between or with cells of interest, and is considered a core region which may affect the secondary structure of the present peptides and assists in maintaining the molecular characteristics of the other regions as described below.

In the present disclosure, the first region or the peptide of formula I may comprises a plurality of the unit composed of 5 amino acids, and each unit may be identical or different when two or more units are included. The number of units included in the present peptides may be various and determined in consideration of the functionalization of interest of the present peptide such as for use in the preparation, storage or delivery, or in consideration of the effects or various applications as described hereinafter. For example, in formula I, n may be 1 to 10, 1 to 9, 1 to 7, 1 to 6, 1 to 5, 1 to 4, 1 to 3, 1 to 2, or 1.

In the present disclosure, one or more of the first region or the peptide of formula I composed of 5 amino acids may be comprised in the present peptides and when more than one is present, each one may be identical or different. The number of formula I which may be included in the present peptides may be various and determined in consideration of the functionalization of interest of the present peptide such as for use in the preparation, storage or delivery, or in consideration of the effects or various applications as described hereinafter. For example, in formula I, n may be 1 to 10, 1 to 9, 1 to 7, 1 to 6, 1 to 5, 1 to 4, 1 to 3, 1 to 2, or 1.

In other embodiment, in formula I, X¹ is any amino acids, particularly polar non-charged amino acids, more particularly S, T, C, P, N or Q.

In other embodiment, in formula I, X², X³ and X⁴, which may be identical or different, and each are L, V, I, E G or A. The sequence of X²-X³-X⁴ are for example AAA, EEE, LVG, LVA, LVL, LVV, LLA, LLL, or LLV and the like without being limited thereto. In other embodiment, the formula I may be X¹-LVV-X⁵, X¹-AAA-X⁵ or X¹-EEE-X⁵.

In one embodiment, the sequence of formula I is represented by QLVVK (SEQ ID NO: 1), QEEEK (SEQ ID NO: 2), QAAAK (SEQ ID NO: 3), NLVVK (SEQ ID NO: 4), SLVVK (SEQ ID NO: 5), QVVVK (SEQ ID NO: 70), QIVVK (SEQ ID NO: 71), QAVVK (SEQ ID NO: 72), QLLVK (SEQ ID NO: 73), QLIVK (SEQ ID NO: 74), QLAVK (SEQ ID NO: 75), QLVLK (SEQ ID NO: 76), QLVIK (SEQ ID NO: 77), QLVAK (SEQ ID NO: 78), or QLVVR (SEQ ID NO: 79) without being limited thereto.

In other aspect, the present disclosure relates to a peptide of formula II or a peptide of formula I further comprising formula II as below having the biological property as disclosed herein:

[X⁶ - X⁷ - X⁸ - X⁹ - X¹⁰ - X¹¹]ₙ,

in formula II,
X⁶ is any one of F, Y and W,
X⁷ is K or R,
X⁸ is any one of A, M and I
X⁹ is any one of L, M and G,
X¹⁰ is any amino acid,
X¹¹ is any one of C, S and T,
wherein at least one group of (X⁸ and X⁹) and (X¹⁰ and X¹¹) may be absent, wherein n is 1 or 2, the amino acids are natural or non-natural D- or L- form or its derivatives.

The peptide of formula II may be linked to an amino terminal (N-term) or a carboxy terminal (C-term), or both of N- and C-term of the peptide of formula I.

According to the present disclosure, the peptide of formula II, referred to as a second region/domain, imparts a hydrophilic property to the present peptides enabling easy dissociation and may form an alpha helix as a secondary structure when it is present as a single molecule in combination with the peptide of formula I.

In one embodiment, at least one of the peptide of formula I and formula II each may be included in the present peptide in various arrangements. For example, the present peptides may include a peptide in which at least one of peptide of formula I being connected to at least one of peptide of formula II, including for example a peptide of formula I-II, a peptide of formula II-I, a peptide of formula I-I-formula II-II, or in which at least two of consecutively connected peptides of formula I and II are further linked, including for example a peptide of formula I-II-I-II, or formula I-II-I, or formula II-I-II. The order may be changed.

In one embodiment of the present disclosure, the peptide of formula II is represented by FRALPC (SEQ ID NO: 6), FREEPC (SEQ ID NO: 7), FRVVPC (SEQ ID NO: 8), FEALPC (SEQ ID NO: 9), YRALPC (SEQ ID NO: 10), WRALPC (SEQ ID NO: 11), FRALP (SEQ ID NO: 12), FRAL (SEQ ID NO: 13), or FRPC (SEQ ID NO: 14) or FR.

In other aspect of the present disclosure, the peptide of formula I, II or the peptide including both formula I and II may further comprise a peptide of formula III: X¹²₁₋₁₅ at its Nor C-term, including such as formula III-I-II, wherein X¹² is positively or negatively charged amino acids. When the present polypeptide starts with formula III, the first amino acid may be positively or negative charged, which are also encompassed in the present disclosure,

In other aspect of the present disclosure, the peptide of formula I, II or the peptide including both formula I and II may further comprise a peptide of formula III called a third region: X¹²₁₋₁₅ at its N- or C-term, including wherein X¹² is positively or negatively charged amino acids.

X¹² is positively or negatively charged amino acids.

In one embodiment, in formula III, the positively charged amino acid is K or R, or F, K or R.

In other embodiment, in formula III, the negatively charged amino acid is D or E.

In other embodiment of the present disclosure, it is provided a peptide of formula IV or a composition comprising the same having the biological activity of promoting bone regeneration or bone formation.

[Formula IV] [X¹²]₁₋₁₅-[X¹-X²-X³-X⁴-X⁵]ₘ₋[X⁶-X⁷-X⁸-X⁹-X¹⁰-X¹¹]ₙ,

The definition for each of the peptide comprised in the peptide of formula IV may be referred to as described hereinbefore.

Or In Formula IV,
In -[X¹-X²-X³-X⁴-X⁵]ₘ
X¹ is any amino acid,
X², X³ and X⁴, which may be identical or different, are each L, V, I, E, G or A, at least one of X², X³ and X⁴ may be absent, and
X⁵ is K or R,
m is an integer from 1 to 5, if m is 2 or more, each polypeptide may be identical or different;
In [X⁶-X⁷-X⁸-X⁹-X¹⁰-X¹¹]ₙ,
[X⁶-X⁷-X⁸-X⁹-X¹⁰-X¹¹]ₙ may be absent, or
X⁶ is any one of F, Y and W,
X⁷ is K or R,
X⁸ is any one of A, M and I
X⁹ is any one of L, M and G,
X¹⁰ is any amino acid, and
X¹¹ is any one of C, S and T,
wherein at least one group of (X⁸ and X⁹) and (X¹⁰ and X¹¹) may be absent, wherein n is 1 or 2, the amino acids are natural or non-natural D- or L- form or its derivatives.

In one embodiment, at least two peptides of Formula IV may be linked together for example in the N to C direction.

In other embodiment, any one of amino acid of X¹ of Formula IV may be polar S, T, C, P, N or Q, X²-X³-X⁴ may be AAA, EEE, LVG, LVA, LVL, LVV, LLA, LLL, or LLV, and [X¹-X²-X³-X⁴-X⁵] of Formula IV may be represented by QLVVK (SEQ ID NO: 1), QEEEK (SEQ ID NO: 2), QAAAK (SEQ ID NO: 3), NLVVK (SEQ ID NO: 4), SLVVK (SEQ ID NO: 5), QVVVK (SEQ ID NO: 70), QIVVK (SEQ ID NO: 71), QAVVK (SEQ ID NO: 72), QLLVK (SEQ ID NO: 73), QLIVK (SEQ ID NO: 74), QLAVK (SEQ ID NO: 75), QLVLK (SEQ ID NO: 76), QLVIK (SEQ ID NO: 77), QLVAK (SEQ ID NO: 78), or QLVVR (SEQ ID NO: 79), [X⁶-X⁷-X⁸-X⁹-X¹⁰-X¹¹] of Formula IV may be represented by FRALPC (SEQ ID NO: 6), FREEPC (SEQ ID NO: 7), FRVVPC (SEQ ID NO: 8), FEALPC (SEQ ID NO: 9), YRALPC (SEQ ID NO: 10), WRALPC (SEQ ID NO: 11), FRALP (SEQ ID NO: 12), FRAL(SEQ ID NO: 13), FRPC (SEQ ID NO: 14) or FR.

In other embodiment, the amino acid sequence of the peptides comprised in the above definition may be represented by SEQ ID NO: 15 to 33, or 37 to 69.

In other embodiment, in peptide of Formula IV, X¹ is Q, N, S, T, P or C, X2, X3 and X4, which may be identical or different, are each L, V, I or A, X⁵ is positively charged K or R, [X⁶-X⁷-X⁸-X⁹-X¹⁰-X¹¹] in which at least one group of (X⁸ and X⁹) and (X¹⁰ and X¹¹) may be absent, is FRALPC (SEQ ID NO: 6), FREEPC (SEQ ID NO: 7), FRVVPC (SEQ ID NO: 8), FEALPC (SEQ ID NO: 9), YRALPC (SEQ ID NO: 10), WRALPC (SEQ ID NO: 11), FRALP (SEQ ID NO: 12), FRAL(SEQ ID NO: 13), or FRPC (SEQ ID NO: 14), or FR, X¹² is positively or negatively charged amino acids, m and n is 1 or 2, if m or n is 2, each polypeptide may be identical or different, the amino acids are natural or non-natural D- or L-form amino acids.

In this case, X²-X³-X⁴ may be LVV, AAA, LVA, LVL, LLA, LLL, or LLV.

Further in this case the [X1-X2-X3-X4-X5] may be QLVVK (SEQ ID NO: 1), QAAAK (SEQ ID NO: 3), NLVVK (SEQ ID NO: 4), SLVVK (SEQ ID NO: 5), QVVVK (SEQ ID NO: 70), QIVVK (SEQ ID NO: 71), QAVVK (SEQ ID NO: 72), QLLVK (SEQ ID NO: 73), QLIVK (SEQ ID NO: 74), QLAVK (SEQ ID NO: 75), QLVLK (SEQ ID NO: 76), QLVIK (SEQ ID NO: 77), QLVAK (SEQ ID NO: 78), or QLVVR (SEQ ID NO: 79) .

In one embodiment, the present peptide meeting the above condition has the amino acid sequence represented by SEQ ID NO: 17, 19, 20, 21, 24, 25, 26, 27, 30, 31, 32, 33, 37, 38, 39, 40. 41, 42, 43, 44, 46. 47, 48, 50, 51, 52 or 54.

In other aspect of the present disclosure, it is provided a peptide having the activity for promoting bone regeneration or bone formation or a composition comprising the same, particularly the peptide having the amino acid sequence represented by SEQ ID NO: 15 to 69.

The polypeptides having various amino acid sequences according to the present invention are generated in consideration of the experimental data disclosed herein and the description as to possible modifications of the amino acid residues as described herein below, thus the person of ordinary skill in the art would be able to understand the effect of the present peptide on the bone formation and regeneration without undue experimentation in case where there are no direct experimental results.

The polypeptides according to the present invention are not limited to the above-described sequences, but include biological equivalents thereof. The term biological equivalents refer to polypeptides which contain additional modifications to the amino acid sequences disclosed herein, but have substantially the same or similar activity as the polypeptide disclosed herein.

Such modifications include, for example, a deletion, insertion and/or substitution of one or more residues in the amino acid sequence. The modifications may be determined in consideration of properties of the similarity of side chains such as sizes, charges, hydrophobic or hydrophilicity. Based on the characteristics of the side chains in terms of size, shape and chemical/electrical properties, it is considered that arginine, lysine and histidine are positively charged residue; alanine, glycine, and serine are having similar size of side chains; phenylalanine, tryptophan and tyrosine are having similar structure of side chains. Thus, in consideration of this, arginine, lysine and histidine; alanine, glycine and serine; phenylalanine, tryptophan and tyrosine are considered biologically equivalent.

In one embodiment, the present peptides also include ones having a conservative amino acid substitution(s) in the present peptides. The conservative substitution refers to a substitution without substantially affecting or changing the activity of the original peptides. The substitutions may be for example included at least one.

The conservative amino acid substitutions are known in the art, which may be referred to: Table 1 based on BLOcks Substitution Matrix; Creighton (1984) Proteins. W. H. Freeman and Company (Eds); and Henikoff, S.; Henikoff, J.G. (1992). "Amino Acid Substitution Matrices from Protein Blocks". PNAS 89 (22): 10915-10919. doi:10.1073/pnas.89.22.10915; WO2009012175 A1.

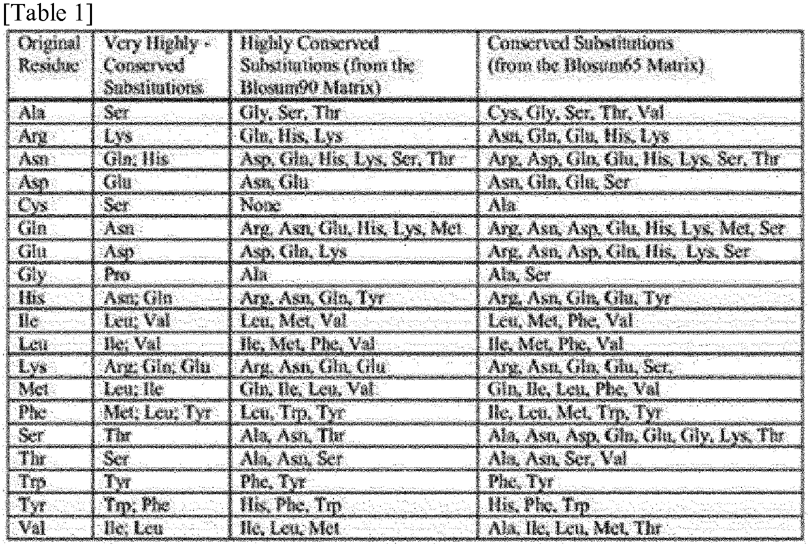

Thus in one embodiment, the present peptides includes ones having the amino acid sequence represented by SEQ ID NO: 1 to 69, and the one having conservative amino acid substitution in the above peptides.

Also when introducing modifications, hydrophobic indices may be considered. Each amino acids is endowed with a unique hydrophobic index according to its hydrophobicity and charges as follows: Isoleucine (+4.5); Valine (+4.2); Leucine (+3.8); Phenylalanine (+2.8); Cysteine/Cystine (+2.5); Methionine (+1.9); Alanine (+1.8); Glycine (-0.4); Threonine (-0.7); Serine (-0.8); Tryptophan (-0.9); Tyrosine (-1.3); Proline (-1.6); Histidine (-3.2); Glutamate (-3.5); Glutamine (-3.5); Aspartate (-3.5); Asparagine (-3.5); Lysine (-3.9); and Arginine (-4.5).

The hydrophobic indices described as above are useful in imparting proteins with an interactive biological function. It is known that similar biological activities are obtained from substitutions with amino acid having similar hydrophobic index. When modifications are performed in reference to the hydrophobic index, it is preferable to select an amino acid for a substitution having a hydrophobic index difference within ± 2, more preferably ± 1, particularly more preferably ± 0.5.

Also it is known that the substitutions between amino acids having similar hydrophilicity value result in biologically equivalent proteins.

For example, US Patent No: 4,554,101 may be referred, in which hydrophilic values are disclosed as follows: Arginine (+3.0); Lysine (+3.0); Aspartate (+3.0+1); Glutamate (+3.0+1); Serine (+0.3); Asparagine (+0.2); Glutamine (+0.2); Glycine (0); Threonine (-0.4); Proline (-0.5±1); Alanine (-0.5); Histidine (-0.5); Cysteine (-1.0); Methionine (-1.3); Valine (-1.5); Leucine (-1.8); Isoleucine (-1.8); Tyrosine (-2.3); Phenylalanine (-2.5); Tryptophan (-3.4).

Further, the amino acid substitutions that fall within the scope that does not result in a substantial change in the biological characteristics compared to a parent protein are known in the art (H. Neurath, R.L.Hill, The Proteins, 3rd Edition, Academic Press, New York, 1979). For example, typical substitutions include Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu and Asp/Gly.

Furthermore, when considering variants having biologically equivalent activities as described above, it is encompassed in the present invention not only the amino acid sequences disclosed herein or nucleic acids encoding the same as described below, also the sequences substantially identical to the sequences disclosed herein. The term "sequences substantially identical" refers to those showing preferably at least 61%, more preferably at least 70%, still more preferably at least 80%, most preferably at least 90% similarity to the sequence disclosed herein, when aligning sequences with the sequence disclosed herein so as to correspond to each other to the highest possible extent and analyzing the aligned sequences using algorithms that are generally used in the art. Methods of alignment of sequences for comparison are well-known in the art. Various programs and alignment algorithms are described in, for example, Smith and Waterman, Adv. Appl. Math. (1981) 2:482; Needleman and Wunsch, J. Mol. Bio. (1970) 48:443; Pearson and Lipman, Methods in Mol. Biol. (1988) 24: 307-31; Higgins and Sharp, Gene (1988) 73:237-44; Higgins and Sharp, CABIOS (1989) 5:151-3; Corpet et al., Nuc. Acids Res. (1988) 16:10881-90; Huang et al., Comp. Appl. BioSci. (1992) 8:155-65 and Pearson et al., Meth. Mol. Biol. (1994) 24:307-31. The NCBI Basic Local Alignment Search Tool (BLAST) (Altschul et al., J. Mol. Biol. (1990) 215:403-10) is available from the NBCI and the like, for use in connection with the sequence analysis programs such as blast, blastp, blasm, blastx, tblastn and tblastx. The BLAST can be accessed at http://www.ncbi.nlm.nih.gov/BLAST/. A description of how to determine sequence identity using this program is available at http://www.ncbi.nlm.nih.gov/BLAST/blast_help.html.

In one embodiment, the C- and/or N-term, particularly C-term may be substituted with an inert group such as NH₂ group to increase the stability of the peptides.

In a further aspect, the present disclosure also relates to nucleotides sequence or nucleic acid molecule encoding the peptides disclosed herein and vectors containing the same and cells comprising the vector. One of ordinary skill in the art would be able to determine the nucleotide sequence from the codon table known in the art and also would be able to select appropriate vectors and cells to which the present nucleotides are cloned and delivered, respectively.

The present peptide or the composition comprising the same have a biological activity of bone regeneration and bone formation in allogenic and xenograft bones. The present peptides do not show heterotropic ossification.

Thus, the peptides of formula I, II, or IV according to the present invention, or compositions comprising them, can be advantageously used for the treatment of various diseases that require bone regeneration or bone formation, or promoting bone regeneration or bone formation promotion.

As used herein, the term "bone regeneration" refers to a healing of the lost bone tissue and is intended to include promotion of osteogenesis or bone formation. Promoting or promotion as used herein refers to shortening the time for increasing the amount or increasing the amount of bone tissue, osteocyte, or bone matrix, and the like, without limitation, and can be confirmed by the therapeutic effect of fracture and the like.

In this respect, bone regeneration or bone formation can be used interchangeably.

Bones are known to be self-healing tissues when a hard environment is provided. Therefore, it is a special tissue that can be recovered by providing a bone-like environment to the region where bone regeneration is needed. Osteoconduction is the technical term that is used to describe a solid environment, that is, the environment where bone can grow well, and processed bone fragments and hard polymers have been developed and used as a medium (implants) for providing such environment.

However, the osteoconductive material injected from the outside does not reach the stimulation level for the complete bone recovery, and also the biological characteristics of the osteoconductive material *in vivo* and the biological mechanism that responds to the osteoconductive material has not been clarified yet. Osteogenic materials derived from bone tissue do not reach the level of overcoming the technical limitations, and BMP-2, 4,7 and 7 used as a osteoinductive material have many limitations as described before.

The peptide according to the present invention is capable of greatly enhancing bone conduction in vivo and promoting the expression of osteoarthritic growth factor, though not limited to this theory. For example, it is believed that promoting cell adhesion promotes bone conduction and, as a result, promotes osteogenic growth factor expression, and promotes various mechanisms for bone regeneration.

Without being intended to be limited by this theory, the present peptides are found to greatly enhance the bone conduction *in vivo,* and thus promoting the expression of osteoinductive growth factors. For example, promoting the cell adhesion leads to the promotion of the bone conduction and, as a result, to the promotion of osteoinductive growth factor expression, and thus promotes various mechanisms for bone regeneration.

In general, osteoconduction just plays a role as a supporter by mimicking the physicochemical properties of bones, and does not function as an active ingredient per se. On the other hand, the peptides of the present invention can function as an effective ingredient in itself, and it can be said that even though the present peptides can enhance the osteoconductivity but are not working as osteoconductive material acting as a support. As a result, according to the present invention, the bone regeneration has been increased just by using a mixture of the osteoconductive material and the present peptides in the absence of the osteoinductve material, which ultimately results in enhancing the osteoconductivity.

The peptides according to the present invention can replace BMP-2 in that it can lead to rapid recovery of bone loss. Peptides according to the present invention exhibit bone regeneration results comparable to or better than BMP-2 in the rate of bone recovery and the amount of bone mass regenerated.

Thus in other aspect, there is provided a pharmaceutical composition comprising the present peptides for promoting bone regeneration and/or bone formation, or for treating disease that requires bone regeneration or bone formation.

As used herein the term "treatment" or "treating" refers to the administration of a composition of the present invention to a mammal to delay, inhibit, alleviate, or eliminate the development of a disease or disease that requires bone regeneration or promotion of bone formation.

As used herein the term "prevention" or "preventing" refers to inhibit the development of a disease or disorder that requires bone regeneration or promotion of bone formation by administering the present composition to a mammal.

The peptide according to the present invention can be used for treating or preventing various diseases that requires an increase in bone density due to bone loss or the increased risk of fracture resulting from the low bone density.

Diseases that require bone regeneration and bone formation promotion include, but are not limited to, fractures, alveolar bone regeneration, arthroplasty, spinal fusion, and bone cysts/bone tumors. Also included are diseases that require a surgical operation such as bone graft because the natural recovery of bone is difficult to be achieved due to the diseases as described above.

Further diseases for which the peptides or compositions according to the present invention may be used include, but are not limited to, closed, open and nonunion fractures; Prophylactic treatment for maximal bone mineral density by closure and open fracture reduction; Promoting bone healing in plastic surgery; Stimulation of bone growth after non-cemented orthopedic surgery and dental implant; Increasing the bone mineral density to the maximum of premenopausal women; Treatment of growth deficiency; Treatment of osteolytic bone disease such as cancer; Periodontal disease and defect treatment and other tooth restoration processes; Increased bone formation during renal cartilage formation; Osteoporosis associated with aging, postmenopausal osteoporosis, glucocorticoid-induced osteoporosis, or occlusive osteoporosis and the like.

Those of skill in the art will be able to determine the delay, inhibition, alleviation, or elimination of a symptom or disorder using a variety of methods known in the art.

The peptides or pharmaceutical compositions of the present invention may be used for the promotion of bone regeneration following congenital, traumatic or surgical resection (for example, cancer treatment) or plastic surgery.

In addition, the peptide of the present invention can be used in combination with any existing bone substitutes, and a higher recovery level can be expected than a sole use of the bone substitutes.

In other embodiments, the peptides according to the present invention may be used in place of or in combination with BMP-2 to treat or prevent various diseases in which BMP-2 is used.

The composition according to the present application may be provided in the form of a pharmaceutical composition.

The pharmaceutical compositions herein may be prepared by employing one or more pharmaceutically or physiologically acceptable carriers with the peptide according to the present invention as an active ingredient.

The term carrier as used herein means a pharmaceutically acceptable carrier, excipient, or stabilizer that is non-toxic to the cell or mammal exposed to the dose and concentration of the composition employed. Examples of such carriers include buffers such as saline, Ringer's solution, buffered saline, phosphate, citrate and other organic acids, antioxidants including ascorbic acid, low molecular weight (less than about 10 residues) polypeptides, proteins such as serum albumin, gelatin or immunoglobulins; hydrophilic polymers such as polyvinylpyrrolidone, amino acids for example glycine, glutamine, asparagine, arginine or lysine, carbohydrate for drug such as monosaccharides, disaccharides and glucose, mannose or dextrin, chelating agents such as EDTA, sugar alcohols such as mannitol or sorbitol, salt forming counter ions such as sodium, and (or) non-ionic surfactants such as twin, polyethylene glycol (PEG) and PLURONICS. The present composition can be administered by various routes. In one embodiment, parenteral administration (for example, intravenous, subcutaneous, intraperitoneal, or local administration), and parenteral administration is particularly preferred. The dosage may vary depending on the condition and the weight of the patient, the severity of the condition and the like.

If necessary, other conventional additives such as an antioxidant, a buffer, and a bacteriostatic agent may be added. In addition, it can be formulated into injection formulations such as aqueous solutions, suspensions, emulsions and the like, pills, capsules, granules or tablets by additionally adding diluents, dispersants, surfactants, binders or lubricants. Or specific antibody or other ligand can be used in combination with the carrier for targeted delivery. Further, it can be advantageously formulated according to the disease or components of interest using the appropriate method in the art or using the method disclosed in Remington's Pharmaceutical Scinece, Mack Publishing Company, Easton PA (latest edition).

The composition of the present invention may be administered parenterally or the specific method of administration may vary depending on the administration route and time, and may be appropriately selected by those skilled in the art.

Formulations for parenteral administration include sterilized aqueous solutions, non-aqueous solutions, suspensions, emulsions, freeze-dried preparations, suppositories, and the like.

Examples of the non-aqueous solvent and suspending agent include propylene glycol, polyethylene glycol, vegetable oil such as olive oil, injectable ester such as ethyl oleate, and the like. As a base for suppositories, witepsol, macrogol, tween 61, cacao paper, laurin, glycerol, gelatin and the like can be used.

The composition according to the invention is administered in a pharmaceutically effective amount. In the present invention, "pharmaceutically or therapeutically effective amount" means an amount sufficient to treat a disease at a reasonable benefit/risk ratio applicable to medical treatment, and the effective dose level will depend on the type, severity, the activity of the drug, the sensitivity to the drug, the time of administration, the route of administration and rate of release, the duration of the treatment, factors including co-administered drugs, and other factors well known in the medical arts. The composition of the present invention can be administered as an individual therapeutic agent or in combination with other therapeutic agents, and can be administered sequentially or simultaneously with conventional therapeutic agents, and can be administered singly or in multiple doses. It is important to take into account all of the above factors and to administer the amount in which the maximum effect can be obtained in a minimal amount without side effects, which can be easily determined by those skilled in the art.

Specifically, the effective amount of the composition according to the present invention may vary depending on the age, sex, and body weight of the patient. In general, 0.01 µg to 100 mg, preferably 0.01 µg to 10 mg per 1 kg of body weight, which may be administered daily, very other day, or one to three times a day. However, the dosage may be varied depending on the route of administration, the severity of disease, sex, weight, age, etc. Therefore, the dosage is not limited to the scope of the present invention by any means.

The composition of the present invention may further comprise, in addition to the peptide according to the present invention, one or more active ingredients exhibiting the same or similar functions, or a compound that maintains / increases the solubility and / or absorbency of the active ingredient.

In addition, the composition of the present invention can be used alone or in combination with surgery, drug therapy or other methods susing biological response modifiers.

The peptides herein may also be administered in the form of their pharmaceutically active salts. Suitable pharmaceutically active salts include acid addition salts and alkali salts or alkaline earth metal salts. For example, sodium, potassium, lithium, magnesium or calcium salts can be obtained.

The pharmaceutically acceptable salts of the peptides according to the present invention have the desired biological activity of the parent peptide without toxic side effects. Examples of such salts include (a) acid addition salts formed with inorganic acids such as, for example, hydrochloric acid, hydrobromic acid, sulfuric acid, phosphoric acid, nitric acid and the like; and salts formed with organic acids such as acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, naphthalene sulfonic acid, naphthalene disulfonic acid, polygalacturonic acid, and the like; (b) base addition salts formed from polyvalent metal cations such as zinc, calcium, bismuth, barium, magnesium, aluminum, copper, cobalt, nickel, cadmium and the like; or base addition salts formed from organic cation formed from N, N'-dibenzylethylenediamine or ethylenediamine; or (c) a combination of (a) such as a zinc tannin salt and the like and (b).

In another aspect, the present invention also provides a method of promoting bone regeneration or bone formation using the peptide according to the present invention or a composition comprising the same.

In another aspect, the invention also provides a method of bone regeneration or bone formation or treating or preventing disease that requires the promotion of bone formation or regeneration comprising administering an effective amount of the present peptides or the composition comprising the same to a subject in need thereof

The kind of the specific disease, the peptide, the dose, and the administration method included in the present method can be referred to the above.

Subjects for which the pharmaceutical compositions or methods according to the present invention are used include, but are not limited to, mammals including, for example, dogs, cows, horses, primates, and humans.

Hereinafter, the present invention will be described in further detail with reference to examples. It is to be understood, however, that these examples are for illustrative purposes only and are not intended to limit the scope of the present invention.

### EXAMPLES

### EXAMPLE 1. Preparation of the present peptides

The peptides used in the Examples were synthesized by 9-fluorenylmethyloxycarbonyl (Fmoc) method (Lugen Sci, Korea; Peptron, Korea). To confirm the reproducibility of the experimental results using the peptides, identical sets of peptides were independently synthesized 2 or 3 times from Lugen Sci and Peptron, respectively. Consistent results were obtained from the peptides synthesized from different companies and among different batches.

### EXAMPLE 2. Effect of the present peptides on the cell adhesiveness that is a main mechanism of bone regeneration

The following experiments were performed to test the present peptide for promoting bone regeneration using peptide having the sequence represented by SEQ ID NO: 17 in xenograft bone model and in the following Examples unless indicated otherwise.

Using the graft materials having the components as in Table 2, each of the following four groups was grafted on the back of the immunodeficient mice as described below, and newly formed hard tissues were analyzed over 3 weeks and 6 weeks after the graft. The doses used were as follows: Geistlich Bio-Oss: 100 mg; hDPSC (human dental pulp stem cell): 5x10⁶ cells; Peptide according to the invention: 10µg; and BMP-2 (BD Science): 2 µg (Kevin et al., Adv Drug Deliv Rev. 2012; 64: 1277-1291).

**[Table 2]**

| | Group1 | Group2 | Group3 | Group4 |
|---|---|---|---|---|
| | Control: Saline treated (negative) | Peptide treated | BMP-2 treated (positive) | Treated with mixture |
| Bio-OSS | ○ | ○ | ○ | ○ |
| hDPSC | ○ | ○ | ○ | ○ |
| BMP-2 | - | - | ○ | ○ |
| The present peptide | - | ○ | - | ○ |

For the animal experiments, 6-week-old immunocompromised mice (NIH-bg-nu / nuxid, Harlan Sprague Dawley, Indianapolis, IN, USA) were anesthetized and each of the composition as above with 0.5% fibrin gel was inserted in the incision made on the back of the mouse and the incisions were re-sutured. Peptides and BMP-2 were diluted in saline solution and the same volume of saline solution was used as a control. There were no significant changes in body weight of the mice before and after the surgery, and there was no significant difference in the consumption of feed. Before and after the operation, the breeding and management of the experimental animals were performed according to the criteria of the laboratory animal center of Seoul National University.

The mice were sacrificed at 3 and 6 weeks after each composition as described above was administered, and the treated sites were separated and fixed in a 4% para-formaldehyde solution. After decalcification in 10% EDTA solution, the tissues were embedded in paraffin and sectioned for H&E staining and immunostaining. Immunostaining was performed to detect a hard tissue marker, Osteocalcin, in addition to the use of antibody to CD31 for vascular formation analysis. Quantitative analysis of the newly formed hard tissues using H&E staining was performed as previously described from (Lee et al., Biomaterials 2011; 32: 9696-9706).

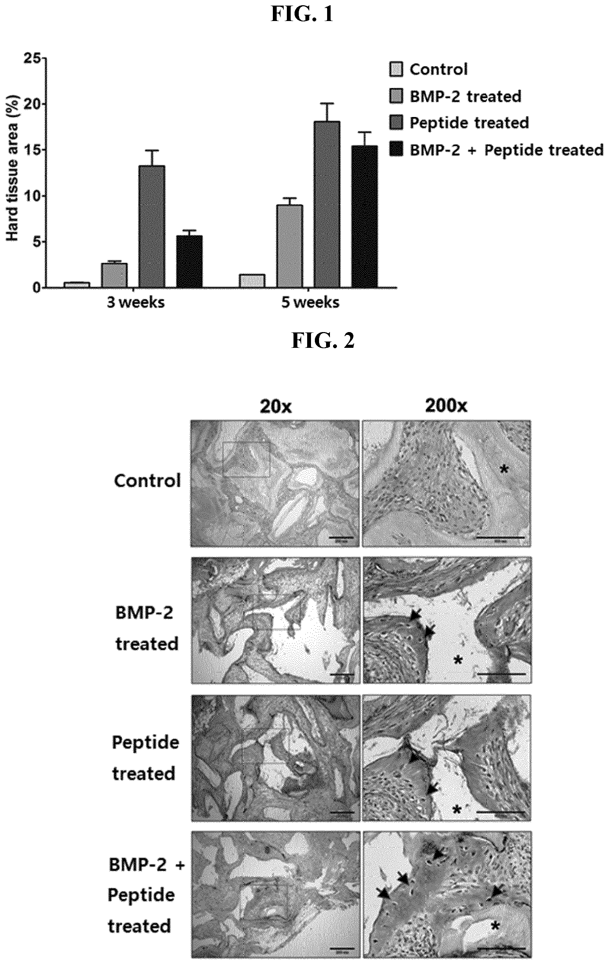

The results showing the increased hard tissue formation by the treatment of the peptide treatment according to the present disclosure are shown in FIG. 1. According to FIG. 1, the hard tissue formation in each experimental condition was analyzed at 3 weeks, and it was confirmed that the amount of hard tissue formed is remarkably increased.by the treatment of the present peptide compared to the others. In the BMP-2 treated group used as a positive control, the hard tissue regeneration was increased compared to the negative control group, but more prominent tissue regeneration was observed in the group treated the present peptide. On the other hand, in the mixed treatment group (peptide + BMP-2), the effect was less than the group treated with the peptide alone treatment but still increased about 1.8 times as compared with the group treated with BMP-2 alone. In the xenograft bone graft model, the peptide treatment alone was able to result in the highest rate of hard tissue development. Analysis of hard tissue formation at 6 weeks showed that the highest level of hard tissue development was still observed in the group treated with the peptide alone and that the hard tissue development in the mixed treated group was at the level of the peptide alone group even though there was a slight difference. In the BMP-2 treated group, hard tissue development was significantly higher than that of the negative control group but lower than that of the present peptide alone or mixed treatment group.

Histological analysis and observation results of the hard tissue by the present peptide treatment group are shown in FIG. 2. FIG. 2 shows the results of immunohistochemical staining of osteocalcin (OC, a marker of bone calcification) to examine the osteogenic tissue of the regenerated tissue. It clearly confirms the tissue development of the osteocyte type form and the distribution pattern of the calcified tissue found in the mature natural bone. Also, when compared with the tissue regeneration by a bone inducing factor BMP-2 treatment, it was confirmed that there is no difference in the characteristics of hard tissue regenerated by the treatment of the present peptide.

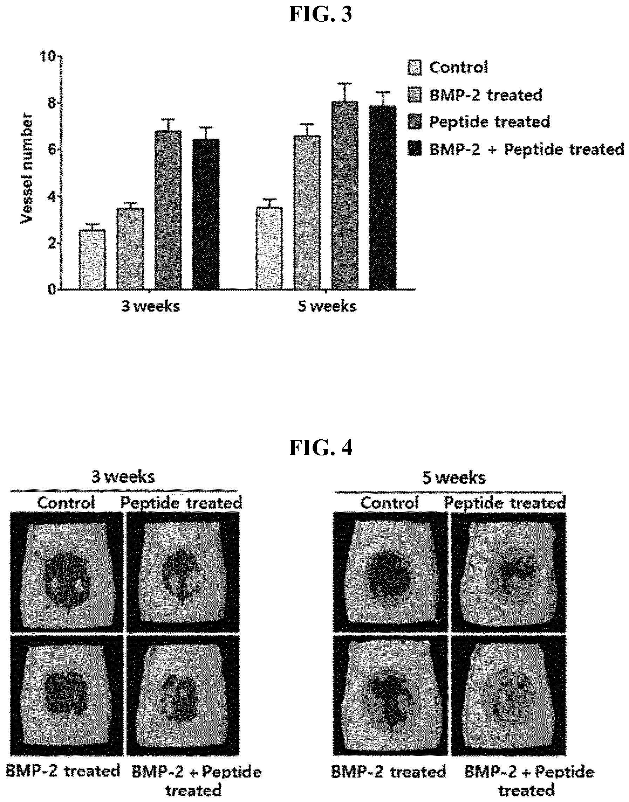

The results of angiogenesis by the peptide treatment according to the present disclosure are shown in FIG. 3. FIG. 3 shows that the angiogenesis or blood supply is well developed in the newly formed hard tissue in the xenograft bone regeneration model. In the field of xenograft bone grafts, the smooth supply of blood is known to have a great influence on the quality of overall tissue regeneration, and it is also a major determinant of the successful tissue regeneration after transplantation in the clinic. Immunohistochemical staining for CD31, a marker of blood cells and blood vessels, showed that angiogenesis is well developed in proportion to the level of hard tissue regeneration (FIG. 1). Particularly in the group treated with composition containing the present peptides showed excellent blood vessel formation as compared to the positive control (BMP-2).

From the above results, it can be seen that the peptide according to the present invention exerts an excellent effect on bone regeneration in a xenograft bone graft model, and thus can be applied to various xenograft bone grafting fields and can be used effectively for rapid bone regeneration overcoming or compensating for the disadvantages of BMP- 2.

### EXAMPLE 3. Promoting bone regeneration in calvarial defect model by the present peptides.

The bone regeneration ability of the peptide according to the present invention was analyzed using calvarial defect model as follows.

First, a graft material having components corresponding to the respective conditions in Table 3 was used on laboratory rats as an animal model was. 8 - week - old male rats were used to avoid errors due to hormonal changes. The breeding and management of the experimental animals were performed according to the criteria of the laboratory animal center of Seoul National University. After preparing the grafts according to the conditions shown in Table 3, a fracture with a particular size was surgically induced in the calvarium of the rat and treated with the grafts. At 3 and 5 weeks after the graft, newly formed bone tissues were examined. Bone-regeneration peptides and BMP-2 were used diluted in saline solution and the same volume of saline solution was used as a control. The doses used were: ACS (absorbable collagen sponge) (Kevin et al., Adv Drug Deliv Rev. 2012; 64: 1277-1291): 8 mm diameter; Peptide according to the invention: 100 µg / ACS disc; rhBMP-2: 2µg / ACS disc

**[Table 3]**

| | Group1 | Group2 | Group3 | Group4 |
|---|---|---|---|---|
| | Control: Saline treated | Peptide treated | BMP-2 treated | Treated with mixture |
| ACS | ○ | ○ | ○ | ○ |
| BMP-2 | - | - | ○ | ○ |
| Peptide | - | ○ | - | ○ |

For animal experiments, calvarial fracture model was prepared using anesthetized experimental rats. The skin of the crown was incised to expose the entire cranium. A diamond-coated trephine drill bit was used to remove the tissue of 8 mm in diameter. After the defect in the calvarim, the prepared ACS was grafted to the site of injury and the incision site was sutured quickly. The size of the damaged tissue, 8 mm in diameter, corresponds to a critical defect in rats where the wound is not well recovered. Deficits defined as serious are known as a good model for evaluating the efficacy of drugs. Surgery was performed carefully to prevent dura mater injury during surgery. Cerebrospinal membrane injury can be judged based on visual observation or excessive bleeding. There was no significant difference in body weight before and after surgery, and there was no significant difference in consumption of feed.

The micro-CT method was used for the analysis and the skulls were removed after sacrificing the rats at 3 and 5 weeks after surgery. After washing the skull with saline solution, they were separated and fixed in 4% para-formaldehyde solution, followed by micro-CT analysis. Quantitative analysis of the newly formed regenerated bone was performed by measuring the volume of calcified tissue within the injured area to assess the degree of bone regeneration.

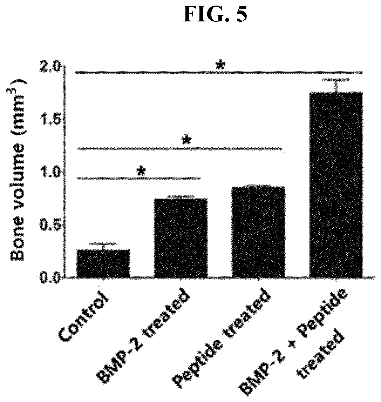

The effect of the present peptide on the bone regeneration is shown in FIGs 4 and 5. FIGs. 4 and 5 respectively show a three-dimensional rendering image and the volume of new bone tissue in the damaged calvarial region. According to the results, it was shown that the bone regeneration in the defect area was significantly increased by the treatment with the present peptide in the calvarial defect model, which is comparable to the result obtained with BMP-2 treatment group showing the induction of bone cell differentiation of progenitor cells. In addition, it was confirmed that when BMP-2 and peptide were mixed, the additive effect was also exhibited. These results indicate that the peptide according to the present invention can be used alone or in combination with a conventional substance capable of promoting bone regeneration to further promote bone regeneration.

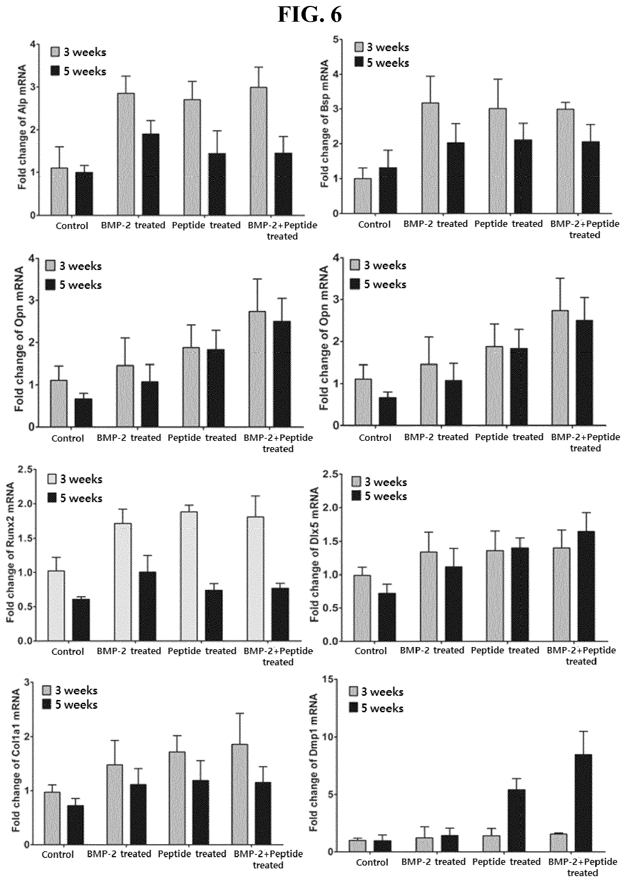

The results of examining the expression of molecular markers for the bone regeneration effect are shown in FIG. 6. This is the quantitative real-time PCR results for each gene indicated in the figure, which were performed on cDNA synthesized from isolated RNA extracted from newly formed tissues at 3 weeks and 5 weeks. The level of expression of the genes was generally higher at 3 week. Higher levels of osteogenesis-related gene expression were detected in the BMP-2, the present peptides, and mixtures treated groups than that of the control. On the other hand, the formation of osteocytes, a final stage of osteoblast differentiation, is a very important criterion for determining the complete maturation of bone tissue. For this, Dmp1 (dentin matrix protein 1) is a major marker of osteocyte. Interestingly, the marker show a significant increase only in the peptide treatment group of the present invention, which can be interpreted that the bone treated with the present peptide have a better bone quality.

### EXAMPLE 4. Analysis whether the present peptide causes ectopic ossification or heterotrophic ossification.

In order to analyze whether the present peptides induce any ectopic ossification, the following experiments were performed.

First, the present peptide or BMP-2 was adsorbed on ACS to prepare a graft material. ACS used was a disc of 5 mm in diameter and the present peptide and BMP-2 were used dissolved in saline solution. BMP-2 was used at the concentration of 2 µg and the present peptide was used at the concentration of 600 µg, which is 300 times that of BMP-2.

For animal experiments, the graft material containing BMP-2 was inserted into an incision made at the left femur muscle of a 6-week-old male mouse, and the graft containing the present peptide was inserted into an incision made at the right femur, which were performed after anesthetizing the mice followed by the sequential incision of the epidermis and fascia of the left and right femur. There was no significant difference in body weight before and after surgery and right before the sacrifice. Further there was no significant difference in the consumption of feed. Eight weeks after the operation, animals were euthanized and micro-CT analysis was performed to observe calcification (ectopic ossification) in the muscle. All 10 animals were used.

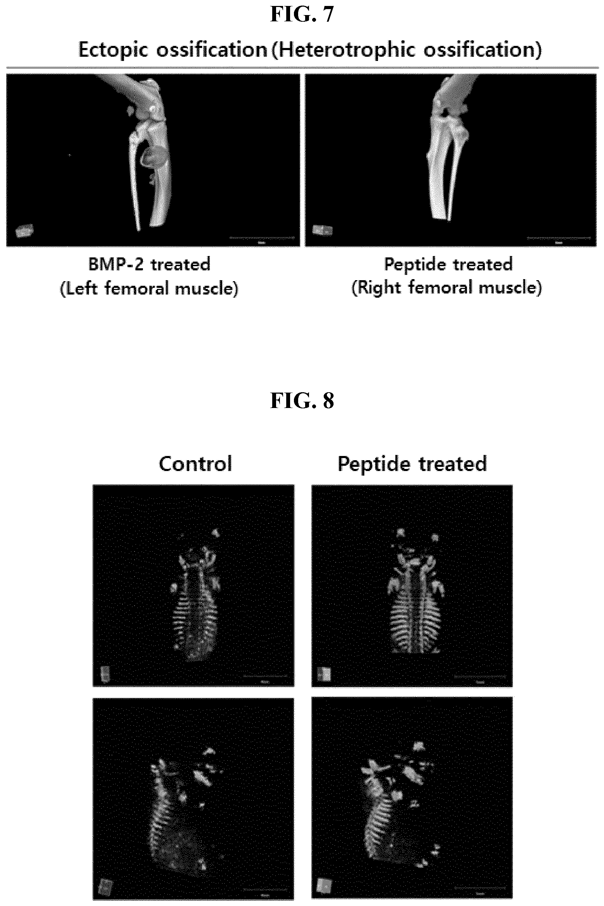

The results are shown in FIG. 7. According to these results, the comparative analysis of the ectopic ossification of each active ingredient inserted into the femur muscles revealed that the left femur into which BMP-2 was inserted showed a distinct ectopic ossification in all the mice treated (n = 10), whereas the right femur into which the present peptide was inserted showed no ectopic ossification in all the mice treated. These results clearly show that the bone regeneration effect by the present peptide is comparable to that of BMP-2, further the present peptide can be used more safely as an active ingredient than BMP-2 because no ectopic ossification was observed with the present peptide even at the high concentration.

### EXAMPLE 5. Analysis of promoting bone formation during embryo development

The effect of the present peptide on promoting the bone formation during the development was analyzed as below.

For this, 10mg/kg of the peptide according to the present invention was injected into the abdominal cavity of a pregnant mouse (C57BL/ 6, 10 weeks old, Orienbio, Korea). Two days (E16.5) after the injection, the mouse was sacrificed and the embryos were removed and analyzed for the bone formation.

From E14.5, the embryo enters into a major stage with vigorous bone formation rate. Bone formation activity was analyzed by micro-CT analysis, total skeletal staining (WSS), and other tissue staining. Specifically, the embryos were fixed in a 4% para-formaldehyde aqueous solution and micro-CT analysis was performed after the operation. Histological analysis was performed after embedding the tissue in paraffin. Tissues were stained with H & E and Alcian blue. In addition, for whole skeletal staining, ethanol fixation followed by Aiizarine red staining for the calcified tissue and Alcian blue staining for the cartilage were performed
The results are shown in FIGs. 8 to 11.

FIG. 8 is the micro-CT analysis result showing that the embryonic bone tissue is in an immature stage. Therefore, the bone mineral density per unit volume in the embryonic development is still weak, and thus the micro-CT analysis does not distinguish the difference in the density of bone tissue from the surrounding soft tissues. Given these characteristics, analysis at the embryonic stage has become an important model for evaluating drugs (active ingredients in new drugs or medical devices) that can positively affect the bone formation. Micro-CT analysis of the bone tissues formed at the embryonic stage after the present peptide treatment revealed that the density of the bone tissue was significantly increased and as a result, the distinct developmental pattern of the skeletal structure was observed. On the other hand, the border between the hard tissue and the surrounding soft tissue was unclear in the control group, indicating the bone tissue is still in immature stage.

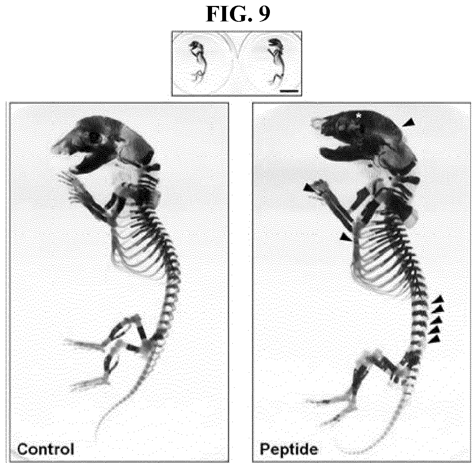

FIG. 9 shows the result of WSS (Whole Skeletal Staining) analysis. The result of Alizarine red staining and Alcian blue staining of the whole 14-day-old embryo bone tissue was confirmed to be similar to those of micro-CT analysis. Alizarine red staining was more pronounced in the group treated with the present peptide indicating that the calcified tissue is more developed in the group treated with the present peptide. This is a result indicating that the bone calcification level is markedly increased by the present peptide resulting in promoting bone formation activity.

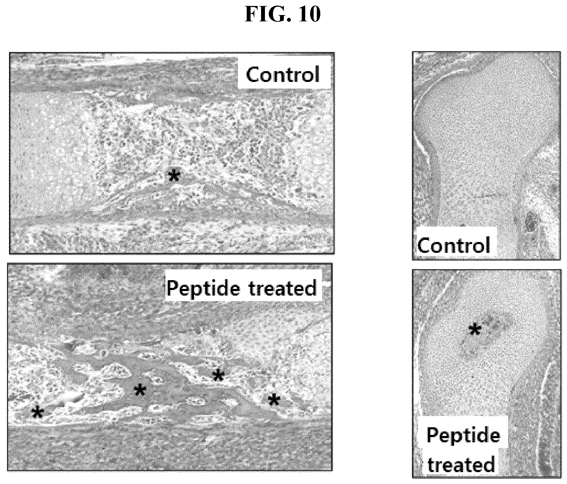

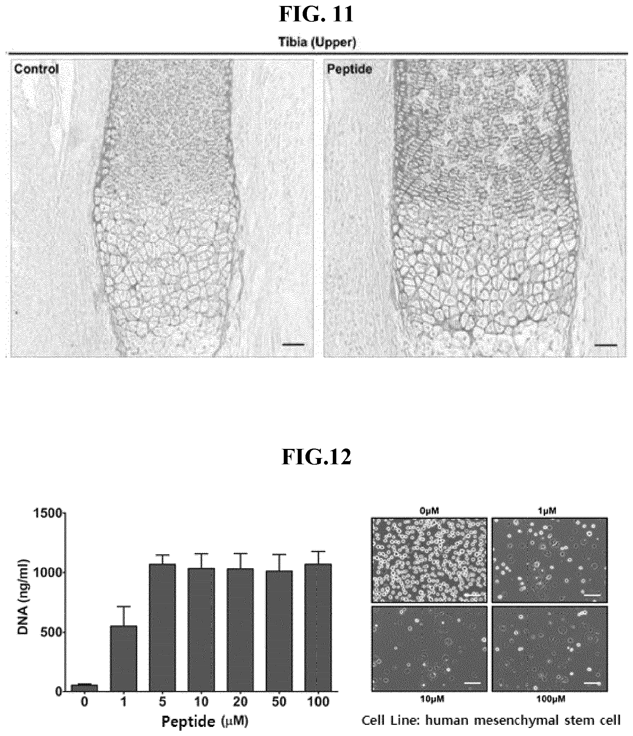

FIGs. 10 and 11 are the results showing the characteristics of calcification and bone formation by staining calcified tissue and cartilage with H&E (FIG. 10) and Alcian blue (FIG. 11) in the hind limb removed from the mice and then fixed in paraffin. In consistent with the WSS analysis, in which the marked development of the calcified tissue was confirmed by the treatment with the present peptides, the calcification was also pronounced in the histological analysis. In other words, the histologic morphology of the bone tissue was more evident in the group treated with the present peptide, and it was also confirmed that the growth in length was also increased in various bone tissue regions. It was also confirmed that a secondary ossification center that is present at the completion stage of bone formation was also observed. In addition, analysis of the histological characteristics of cartilage tissue from H&E staining also showed a marked difference in the group treated by the present peptide, which indicates that the growth of cartilage is also increased. During embryonic development, the differentiation of the bone tissue is preceded by the maturation of the cartilage, followed by the differentiation of the cartilage into the bone tissue. Therefore, it is well known that as the maturity of cartilage increases, it positively affects the bone formation. Thus the Alcian blue staining for the cartilage tissue to test this showed a stronger staining of the extracellular matrix regions (ECM) of chondrocytes, and the histological characteristics (shape of chondrocytes, morphology as the cartilage grows, etc.) with cartilage growth or development were found to be more prominent in the group treated with the present peptide.

### EXAMPLE 6. The promotion of cell adhesion by the present peptides which is a mechanism required for bone formation.

One of the important physiological mechanisms in bone regeneration is osteoconductivity. The technology of increasing the cell adhesion is considered one of the results of increasing the osteoconductivity. In particular, bone grafting can be considered as an overall technology covering the osteoconductivity.

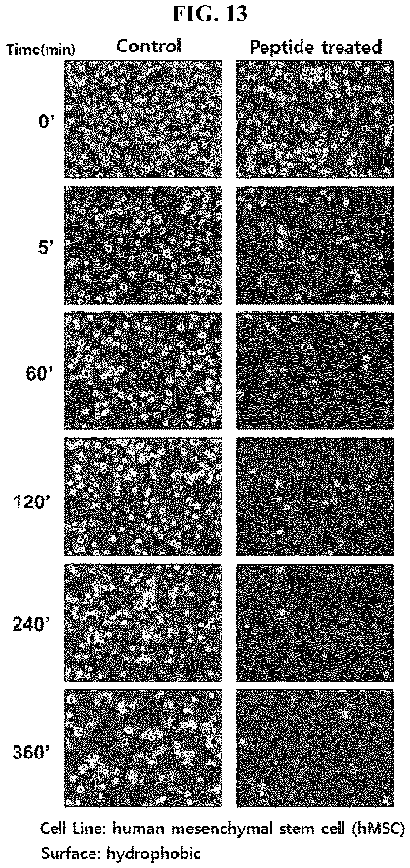

The increases in cell adhesion by the present peptides are shown in FIGs. 12-17. FIG. 12 shows the effect on the cell adhesion to hydrophobic surfaces. Hydrophobic surfaces are generally less responsive to cell adhesion or attachment and however many graft materials have a hydrophobic surface environment. As shown in FIG. 12, in the case of mesenchymal stem cells, which are known to be directly involved in the regeneration of bone tissue in human, the addition of the present peptides effectively induced the cell adhesion. Significant levels of adhesion are observed at the minimum of 1 µM or more. Furthermore, with the addition of 10 µM of the peptides, the appearance of the cell adhesion over time was observed under an optical microscope as shown in FIG. 13. As a result, in the control group, most of the cells were maintained unattached to the surface even after 240 min. In contrast, in the cells treated with the present peptides, it was observed that more than 50% of the cells recognize the surface and thus attach to the surface from as early as 5 min after the treatment. This result indicates that the present peptides can be advantageously used for various graft materials such as surface-treated materials by halogens and thus being rendered not prone to cell attachment, in order to facilitate the cell attachment to the material in vivo and also to promote the interaction between the graft material and the tissues.

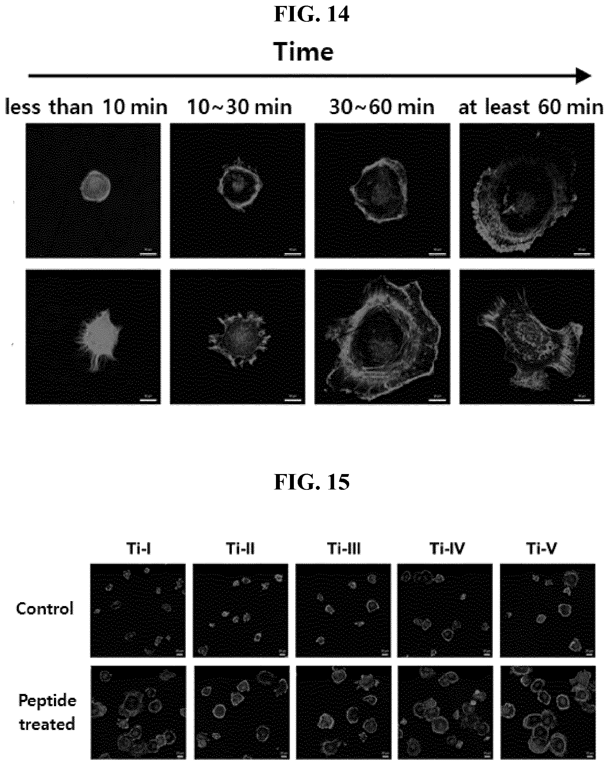

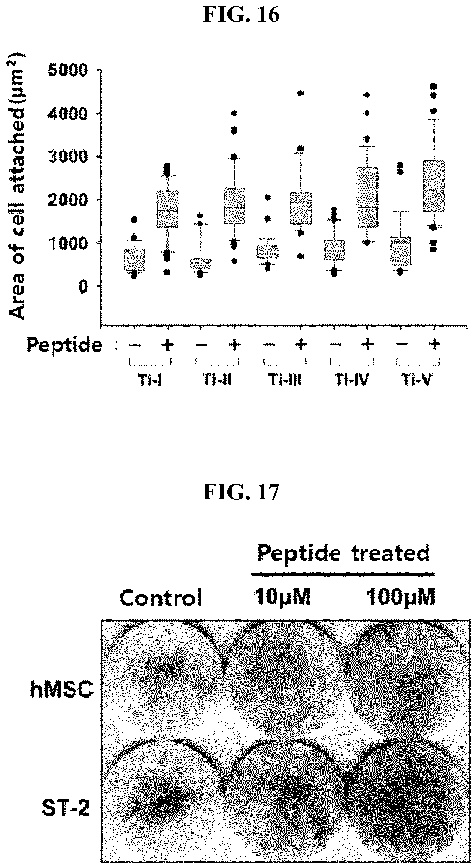

FIG. 14 shows the result of analyzing the cell attachment on a titanium surface, which is one of the most representative materials implanted into a human body. For this, the cells were stained with rhodamine phalloidin and DAPI and examined under confocal microscope. As a cell line for this study, osteoblasts that react relatively well to titanium materials were used. The morphological pattern was more distinct in the peptide-treated group and further the protrusion phenomenon of the cells was very apparent from the beginning in the peptide-treated group. Adhesion of the osteoblasts on titanium surface reached a complete maturation stage in about 60 minutes when the peptide of the present invention was present, whereas the control cells still retained its round shape even after 60 minutes. When the intracellular actin was stained, the cortical actin was observed still at a high level. These results indicate that a large region of cell distribution can be expected in a shorter time when cells are exposed to the peptide of the present invention. The analysis results are shown in FIGs. 15 and 16. FIG. 15 shows the results of observing the adhesion pattern of osteogenic precursor cells on the various titanium surfaces used in orthopedic surgery each having slight differences in the components. There was a clear difference between the present peptide treated group and the control group. In the peptide treated group, wide and dynamic distribution pattern of the cells can be seen and the surface area occupied by the cells was also more extensive. The actual area occupied by each cell was measured and was represented as a box plot in FIG. 16. In the case of the control group, the area of 500-1,000 µm² was obtained while the area of the peptide treatment group was 1,500-3,000 µm². In other words, the area covered by the cells treated with the present peptide treated group was about three times larger in average. The results show that the area attached by the cells at least in the case of titanium implants is at least three times greater than that of the control, and this result can lead to a significant level of difference in the bone fusion rate. This is the reason for the increased bone formation by the peptide of the present invention in the xenograft bone model and calvarial regeneration model as described above. In order to observe the surface attachment patterns of osteoblast or the cells that can differentiate into bone cells on the surface of the graft material, the cells were divided into a control group and the peptide treatment groups and cultured on a zirconia surface having a diameter of 10 mm in size for 72 hours. After culturing, the cells were stained for ALP (alkaline phosphotase, bone marker enzyme) to confirm the cell distribution. The results are shown in FIG. 17. In the control group, the tendency was similar in both the mesenchymal stem cells (hMSC) and the bone marrow-derived undifferentiated stem cells (ST-2), and the distribution of the cells were restricted to a particular region rather than overall distribution. On the other hand, it was observed that the cells of the peptide treated group were uniformly distributed covering most of the areas of the graft material. This implies that the activation of the attachment mechanism at an early stage significantly and positively affects the interaction between the cells and the graft material. However this mechanism is not limited just to the externally applied implants. Rather it can also be applied to promote the bone regeneration where the use of external implants are not required when the peptides are able to attach to the bone tissues just like the present peptides because the bone tissue itself is considered a structure having a perfect osteoconductivity and the implants are the ones that has been developed to have such an osteoconductivity.

### EXAMPLE 7. The effect of the present peptides on the bone regeneration

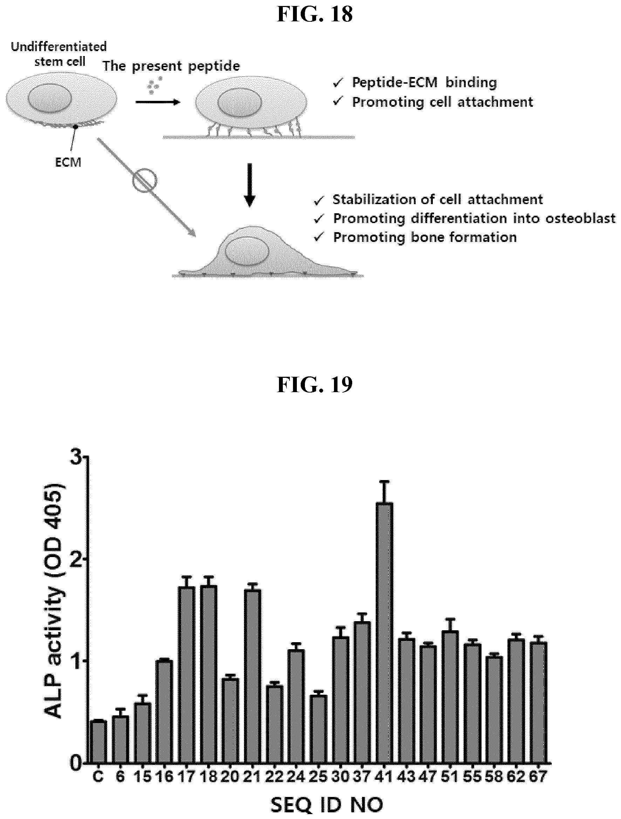

As described above, on the surface of hard structures such as bones or on the surface where cells are difficult to attach to, the peptide of the present invention can promotes the cell adhesion which is a key mechanism for the regeneration. Particularly, it was also found that the osteocyte differentiation can also be promoted by increasing the cell adhesion through binding to ECM on the surface of bone having a high degree of hardness (see FIG. 18). Therefore, in this Example, various adhesive peptides developed and disclosed in the present disclosure were used to test their activity for promoting the osteocyte differentiation. For this, each peptide was mixed with undifferentiated stem cells, which was then cultured on a titanium osteoconductive surface, and analyzed the cells for the differentiation into osteocytes. Results are shown in FIG. 19. Two x 10⁴ cells / ml treated with trypsin and mixed with peptide (10 µM) were cultured on a dental titanium disk having a diameter of 10 mm in size for 6 hours, after which the medium was replaced with osteoblast differentiation medium and the cells were cultured for 48 hours. Then the discs were recovered and the cells were lysed and ALP (Alkaline phosphatase) activity was measured. As shown in the results, a higher level of ALP activity was observed in all the peptides that induce cell adhesion compared to the control (C). In particular, the peptide having SEQ ID NO: 41 [(R) 15-QLVVKFRALPC] positively charged at the N-terminus containing 15 arginine residues showed the highest ALP activity.

## Claims

1. A polypeptide for use in bone regeneration or bone formation having the formula:
[X¹²₁₋₁₅-[X¹-X²-X³-X⁴-X⁵]ₘ-[X⁶-X⁷-X⁸-X⁹-X¹⁰-X¹¹]ₙ,
wherein
in the -[X¹-X²-X³-X⁴-X⁵]ₘ,
X¹ is any amino acid,
X², X³ and X⁴, which may be identical or different, are each L, V, I, E, G or A, at least one of X², X³ and X⁴ may be absent, and
X⁵ is K or R,
m is an integer from 1 to 5, if m is 2 or more, each polypeptide may be identical or different;
in the [X⁶-X⁷-X⁸-X⁹-X¹⁰-X¹¹]ₙ,
the [X⁶-X⁷-X⁸-X⁹-X¹⁰-X¹¹]ₙ may be absent, or
X⁶ is any one of F, Y and W,
X⁷ is K or R,
X⁸ is any one of A, M and I,
X⁹ is any one of L, M and G,
X¹⁰ is any amino acid, and
X¹¹ is any one of C, S and T,
wherein at least one group of (X⁸ and X⁹) and (X¹⁰ and X¹¹) may be absent, wherein n is 1 or 2,
X¹² is any one of F, K and R, and
the amino acid residue is a synthetic or naturally occurring, D- or L-form, or derivatives thereof.

2. The polypeptide for use in bone regeneration or bone formation of claim 1, wherein the X¹ is any one of S, T, C, P, N and Q.

3. The polypeptide for use in bone regeneration or bone formation of claim 1, wherein the X²-X³-X⁴ is AAA, EEE, LVG, LVA, LVL, LVV, LLA, LLL, or LLV.

4. The polypeptide for use in bone regeneration or bone formation of claim 1, wherein the [X¹-X²-X³-X⁴-X⁵] has the amino acid sequence set forth in QLVVK (SEQ ID NO: 1), QEEEK (SEQ ID NO: 2), QAAAK (SEQ ID NO: 3), NLVVK (SEQ ID NO: 4), or SLVVK (SEQ ID NO: 5), QVVVK (SEQ ID NO: 70), QIVVK (SEQ ID NO: 71), QAVVK (SEQ ID NO: 72), QLLVK (SEQ ID NO: 73), QLIVK (SEQ ID NO: 74), QLAVK (SEQ ID NO: 75), QLVLK (SEQ ID NO: 76), QLVIK (SEQ ID NO: 77), QLVAK (SEQ ID NO: 78), or QLVVR (SEQ ID NO: 79)

5. The polypeptide for use in bone regeneration or bone formation of claim 1, wherein the [X⁶-X⁷-X⁸-X⁹-X¹⁰-X¹¹]ₙ has the amino acid sequence set forth in FRALPC (SEQ ID NO: 6), FREEPC (SEQ ID NO: 7), FRVVPC (SEQ ID NO: 8), FEALPC (SEQ ID NO: 9), YRALPC (SEQ ID NO: 10), WRALPC (SEQ ID NO: 11), FRALP (SEQ ID NO: 12), FRAL(SEQ ID NO: 13), FRPC (SEQ ID NO: 14) or FR.

6. The polypeptide for use in bone regeneration or bone formation of claim 1, wherein at least two of the peptides are linked together.

7. The polypeptide for use in bone regeneration or bone formation of any one of claims 1 to 6, wherein the polypeptide has the amino acid sequence set forth in:
RQLVVK (SEQ ID NO: 15);
FRALPCRQLVVK (SEQ ID NO: 16);
RQLVVKFRALPC (SEQ ID NO: 17);
RQLVVKFRALPCRQLVVKFRALPC (SEQ ID NO: 18);
RQLVVKFRALP(SEQ ID NO: 19);
RQLVVKFRAL(SEQ ID NO: 20);
KQLVVKFRALPC (SEQ ID NO: 21);
RQKFRALPC (SEQ ID NO: 22);
RQEEEKFRALPC (SEQ ID NO: 23);
RQAAAKFRALPC (SEQ ID NO: 24);
RQLVVKFRPC (SEQ ID NO: 25);
RQLVVKFREEPC (SEQ ID NO: 26);
RQLVVKFRVVPC (SEQ ID NO: 27);
RQEEEKFREEPC (SEQ ID NO: 28);
EQLVVEFEALPC (SEQ ID NO: 29);
RQLVVKYRALPC (SEQ ID NO: 30);
RQLVVKWRALPC (SEQ ID NO: 31);
RNLVVKFRALPC (SEQ ID NO: 32);
RSLVVKFRALPC (SEQ ID NO: 33);
RQLVVK-(FRALPC)₂ (SEQ ID NO: 37);
(R)₂-QLVVKFRALPC (SEQ ID NO: 38);
(R)₅-QLVVKFRALPC (SEQ ID NO: 39);
(R)₁₀-QLVVKFRALPC (SEQ ID NO: 40);
(R)₁₅-QLVVKFRALPC (SEQ ID NO: 41).
RQVVVKFRALPC (SEQ ID NO: 42);
RQIVVKFRALPC (SEQ ID NO: 43);
RQAVVKFRALPC (SEQ ID NO: 44);
RQEVVKFRALPC (SEQ ID NO: 45);
RQLLVKFRALPC (SEQ ID NO: 46);
RQLIVKFRALPC (SEQ ID NO: 47);
RQLAVKFRALPC (SEQ ID NO: 48);
RQLEVKFRALPC (SEQ ID NO: 49);
RQLVLKFRALPC (SEQ ID NO: 50);
RQLVIKFRALPC (SEQ ID NO: 51);
RQLVAKFRALPC (SEQ ID NO: 52);
RQLVEKFRALPC (SEQ ID NO: 53);
RQLVVRFRALPC (SEQ ID NO: 54);
RQLVVKFKALPC (SEQ ID NO: 55);
RQLVVEFEALPC (SEQ ID NO: 56);
RQLVVKFRLLPC (SEQ ID NO: 57);
RQLVVKFRILPC (SEQ ID NO: 58);
RQLVVKFRVLPC (SEQ ID NO: 59);
RQLVVKFRELPC (SEQ ID NO: 60);
RQLVVKFRAAPC (SEQ ID NO: 61);
RQLVVKFRAIPC (SEQ ID NO: 62);
RQLVVKFRAVPC (SEQ ID NO: 63);
RQLVVKFRAEPC (SEQ ID NO: 64);
RQEEEEFEEEPC (SEQ ID NO: 65);
RQLVVKFRALXC (SEQ ID NO: 66);
RQLVVKFRALPS (SEQ ID NO: 67);
RQLVVKFRALPT (SEQ ID NO: 68); or
RQLVVKFRALPX (SEQ ID NO: 69).

8. The polypeptide for use in bone regeneration or bone formation of claim 7, wherein the peptide further comprises five to twenty K or R residues at its N-terminus.

9. The polypeptide for use in bone regeneration or bone formation of claim 1,
wherein
X¹ is polar Q, N, S, T, P or C.
X², X³ and X⁴, which may be identical or different, are each hydrophobic L, V, I, or A,
X⁵ is positively charged K or R,
in the [X⁶-X⁷-X⁸-X⁹-X¹⁰-X¹¹]ₙ,
at least one group of (X⁸ and X⁹) and (X¹⁰ and X¹¹) may be absent, and has the amino acid sequence set forth in FRALPC (SEQ ID NO: 6), FREEPC (SEQ ID NO: 7), FRVVPC (SEQ ID NO: 8), FEALPC (SEQ ID NO: 9), YRALPC (SEQ ID NO: 10), WRALPC (SEQ ID NO: 11), FRALP (SEQ ID NO: 12), FRAL(SEQ ID NO: 13), FRPC (SEQ ID NO: 14) or FR.
X¹² is positively charged K or R.
m and n are each 1 or 2, when m or n is 2, each amino acid sequence may be same or different, the amino acid is a synthetic or naturally occurring D- or L-form.

10. The polypeptide for use in bone regeneration or bone formation of claim 9, wherein the X²-X³-X⁴ is LVV, AAA, LVA, LVL, LLA, LLL, or LLV.

11. The polypeptide for use in bone regeneration or bone formation of claim 9, wherein the [X¹-X²-X³-X⁴-X⁵] is represented by any one of the following amino acid sequence: QLVVK (SEQ ID NO: 1), QAAAK (SEQ ID NO: 3), NLVVK (SEQ ID NO: 4), SLVVK (SEQ ID NO: 5), QVVVK (SEQ ID NO: 70), QIVVK (SEQ ID NO: 71), QAVVK (SEQ ID NO: 72), QLLVK (SEQ ID NO: 73), QLIVK (SEQ ID NO: 74), QLAVK (SEQ ID NO: 75), QLVLK (SEQ ID NO: 76), QLVIK (SEQ ID NO: 77), QLVAK (SEQ ID NO: 78), or QLVVR (SEQ ID NO: 79).

12. The polypeptide for use in bone regeneration or bone formation of claim 9, wherein the polypeptide is represented by any one of the following amino acid sequence:
RQLVVKFRALPC (SEQ ID NO: 17);
RQLVVKFRALP(SEQ ID NO: 19);
RQLVVKFRAL(SEQ ID NO: 20);
KQLVVKFRALPC (SEQ ID NO: 21);
RQAAAKFRALPC (SEQ ID NO: 24);
RQLVVKFRPC (SEQ ID NO: 25);
RQLVVKFREEPC (SEQ ID NO: 26);
RQLVVKFRVVPC (SEQ ID NO: 27);
RQLVVKYRALPC (SEQ ID NO: 30);
RQLVVKWRALPC (SEQ ID NO: 31);
RNLVVKFRALPC (SEQ ID NO: 32);
RSLVVKFRALPC (SEQ ID NO: 33);
RQLVVK-(FRALPC)2 (SEQ ID NO: 37);
(R)₂-QLVVKFRALPC (SEQ ID NO: 38);
(R)₅-QLVVKFRALPC (SEQ ID NO: 39);
(R)₁₀-QLVVKFRALPC (SEQ ID NO: 40);
(R)₁₅-QLVVKFRALPC (SEQ ID NO: 41);
RQVVVKFRALPC (SEQ ID NO: 42);
RQIVVKFRALPC (SEQ ID NO: 43);
RQAVVKFRALPC (SEQ ID NO: 44);
RQLLVKFRALPC (SEQ ID NO: 46);
RQLIVKFRALPC (SEQ ID NO: 47);
RQLAVKFRALPC (SEQ ID NO: 48);
RQLVLKFRALPC (SEQ ID NO: 50);
RQLVIKFRALPC (SEQ ID NO: 51);
RQLVAKFRALPC (SEQ ID NO: 52); or
RQLVVRFRALPC (SEQ ID NO: 54).

13. The polypeptide for use in bone regeneration or bone formation of claim 12, wherein the peptide further comprises five to twenty K or R residues at its N-terminus.

14. Use of the peptide having an amino acid sequence represented by any one of SEQ ID NO: 15 to 69 for promoting bone formation or bone regeneration.

15. The polypeptide for use in bone regeneration or bone formation of any one of claims 1 to 14, wherein the N- or C-terminus of the peptides are substituted with non-reactive group.

16. The polypeptide for use in bone regeneration or bone formation of any one of claims 1 to 15, wherein the peptides are provided in the form of a pharmaceutical composition.

17. The polypeptide for use in bone regeneration or bone formation of any one of claims 1 to 15, wherein the peptides are used for treating or preventing a disease that requires an increase in the bone density due to bone loss, the disease includes fracture, alveolar bone regeneration, arthroplasty, spine fusion or bone cyst.

18. The polypeptide for use in bone regeneration or bone formation of claim 16, wherein the composition is used for treating or preventing a disease that requires an increase in the bone density, the disease includes fracture, alveolar bone regeneration, arthroplasty, spine fusion or bone cyst.

19. A method of promoting bone formation or bone regeneration by administering the polypeptide of any one of claims 1 to 15 or the composition of claim 16 to the subject in need thereof.

20. The method of claim 19, wherein the method is used to treat or prevent a disease that requires an increase in the bone density due to bone loss and the disease includes fracture, alveolar bone regeneration, arthroplasty, spine fusion or bone cyst.
